# EUROPEAN PATENT APPLICATION

(11) **EP 2 330 182 A1**
(43) Date of publication of application: **08.06.2011**
(21) Application number: 09811604.9
(22) Date of filing: 08.09.2009
(51) Int. Cl.: C12M 3/00, C12N 1/00

(54) **SPHEROID COMPOSITE, SPHEROID-CONTAINING HYDROGEL AND PROCESSES FOR PRODUCTION OF SAME**

(30) Priority: 08.09.2008 JP 2008230224; 08.09.2008 JP 2008230223
(71) Applicant: Tokyo University Of Science Educational Foundation Administrative Organization, Shinjuku-ku Tokyo 162-8601 (JP)
(72) Inventor: OTSUKA, Hidenori, Tokyo 162-8601 (JP); SATOMI, Tomomi, Tokyo 179-0081 (JP); UENO, Koji, Tsukuba-shi Ibaraki 300-2622 (JP); YAMAMOTO, Miyabi, Tokyo 171-0044 (JP); NAKASONE, Yuichi, Tokyo 112-0005 (JP); AKASHI, Kyoko, Narashinoshi Chiba 275-0026 (JP)
(74) Representative: Vossius & Partner
(86) International application number: PCT/JP2009/065641
(87) International publication number: WO 2010/027081

(57) **Abstract**

A spheroid composite includes: a substrate including a cell-adhesive porous base material and plural hydrophilic regions and hydrophobic regions that are disposed on the porous base material and formed by curing a photosensitive composition, wherein the photosensitive composition includes a branched polyalkylene glycol derivative having three or more polyalkylene glycol groups, each having a polymerizable substituent at a terminal thereof, and a tri- or higher-valent linking group that binds to the polyalkylene glycol groups; and spheroids formed in the hydrophobic regions on the substrate, the plural spheroids having a uniform size. A spheroid-containing hydrogel, which includes a hydrogel and two or more spheroids having a uniform size with a diameter of from 70 µm to 400 µm that are disposed in the hydrogel in such a manner that the two or more spheroids do not contact each other, can favorably maintain the function of the plural spheroids contained within the hydrogel.

## Description

### TECHNICAL FIELD

The present invention relates to a spheroid composite and a spheroid-containing hydrogel, and a method of producing the same.

### BACKGROUND ART

General cell cultures, which are two-dimensional plate cultures, are significantly different from biological organs in which many cells are three-dimensionally aggregated, in terms of not only the tissue form but also functional expression. For this reason, in recent years, an attempt to three-dimensionally culture tissue cells of animals including humans and to reconstruct structures that resemble biological organs by using the cultured cells has begun to be studied. As a three-dimensional cell culturing method to be employed therefor, a method of three-dimensionally culturing cells embedded in a collagen gel, and a spheroid formation method are known (see, for example, Patent Document 1). Further, a liquid permeable cell culture support in which a liquid-permeable thread has been proposed (see, for example, Patent Document 2).

Spheroid formation techniques that have been reported include a technique of forming a spheroid having a controlled size by inoculating a determined number of cells in a 96-well plate having a cell-adhesive U-shaped bottom (see, for example, Non-patent Document 1).
Further, a method of forming a spheroid in a gelling material that shows sol-gel transition is known (see, for example, Patent Document 3).
Patent Document 1: Japanese Patent Application Laid-Open (JP-A) No. 7-79772
Patent Document 2: JP-A No. 7-298876
Patent Document 3: JP-ANo. 8-140673
Non-patent Document 1: Yamauchi et al. J. Reprod. Dev. 47 (2001) 165-171

### DISCLOSURE OF THE INVENTION

### Technical Problem

However, in the cases of the collagen gel culture method that is a conventional three-dimensional cell culture method and the spheroid formation method described in Patent Document 1, there is a problem in that it becomes difficult to supply nutrients to inner cells as the three-dimensional structure of the cultured cells becomes larger. At the same time, cell metabolites secreted by the cells (beneficial physiologically-active substances and harmful waste substances) cannot be discharged to the outside, in some cases. Thus, in the case of the three-dimensional cell structures constructed by conventional methods, the inner cells were necrotized as the culture time increases in some cases.
Further, the culture support described in Patent Document 2 had a problem in that the cells adhere to all over the support to provide scaffolds for the cells, which causes the three-dimensional aggregates of the cells to spatially aggregate; therefore, it is difficult to culture the cells for a long time while maintaining the cell functions at high level.

The method described in Non-patent Document 1 had a problem of quite low efficiency of spheroid formation per culture surface area. With the method described in Patent Document 3, it is difficult to control the sizes of the individual spheroids formed in the gel, and it is difficult to form an assembly of uniformly-sized spheroids.

An object of the present invention is provision of a spheroid composite including plural spheroids having a uniform size and disposed on a porous base material and an efficient production method thereof, and a multilayer spheroid composite formed by the spheroid composites disposed one on another in layers.

Another object of the invention is provision of a spheroid-containing hydrogel having excellent function-maintainability and having two or more spheroids contained therein which are disposed in such a manner that the two or more spheroids do not contact each other, and a laminated body thereof, and an efficient method of producing a hydrogel including plural spheroids having a uniform size.

### Solution to Problem

A first aspect of the present invention provides a spheroid composite including:
a substrate including:
   a cell-adhesive porous base material; and
   plural hydrophilic regions and hydrophobic regions that are disposed on the porous base material and are formed by curing a photosensitive composition, wherein the photosensitive composition includes a branched polyalkylene glycol derivative having:
      3 or more polyalkylene glycol groups, each having a polymerizable substituent at a terminal thereof; and
      a tri- or higher-valent linking group that binds to the polyalkylene glycol groups; and
spheroids formed in the hydrophobic regions on the substrate.

The branched polyalkylene glycol derivative preferably has 4 or more polyalkylene glycol groups having polymerization degrees of from 5 to 1,000.
The porous base material preferably includes at least one of a biodegradable compound or an extracellular matrix, and more preferably includes at least one selected from polyglycolic acid, polylactic acid, poly(ε-caprolactone), gelatin, collagen, alginic acid, fibrin, an adhesion protein derived from lectin, polylysine or an adhesive oligopeptide.
The hydrophobic regions are preferably formed in an array on the porous base material.

A second aspect of the invention provides a multilayer spheroid composite including two or more of the spheroid composite, which are disposed one on another in layers.

A third aspect of the invention provides a method of producing a spheroid composite, the method including:
inoculating cells onto a substrate including:
   a cell-adhesive porous base material; and
   plural hydrophilic regions and hydrophobic regions that are disposed on the porous base material and are formed by curing a photosensitive composition, wherein the photosensitive composition includes a branched polyalkylene glycol derivative having:
      3 or more polyalkylene glycol groups, each having a polymerizable substituent at a terminal thereof; and
      a tri- or higher-valent linking group that binds to the polyalkylene glycol groups; and
culturing the inoculated cells to form cultured-cell-derived spheroids in the hydrophobic regions on the substrate.

The branched polyalkylene glycol derivative preferably has 4 or more polyalkylene glycol groups having polymerization degrees of from 5 to 1,000.
The porous base material preferably includes at least one of a biodegradable compound or an extracellular matrix, and more preferably includes at least one selected from polyglycolic acid, polylactic acid, poly(ε-caprolactone), gelatin, collagen, alginic acid, fibrin, an adhesion protein derived from lectin, polylysine or an adhesive oligopeptide.
The porous base material is preferably disposed on a temperature-responsive layer, characteristics of which change in a temperature-responsive manner.
The hydrophobic regions are preferably formed in an array on the porous base material.

A forth aspect of the invention provides a spheroid-containing hydrogel including:
a hydrogel; and
two or more spheroids that have a uniform size with a diameter of from 70 µm to 400 µm, and that are disposed in the hydrogel in such a manner that the two or more spheroids do not contact each other.

The hydrogel preferably includes a polymer compound derived from a macromonomer for forming a hydrogel, wherein the macromonomer has a weight average molecular weight of 10,000 or more, has 4 or more polyalkylene glycol groups, each having a polymerizable substituent at a terminal thereof, and has a tetra- or higher-valent linking group that binds to the polyalkylene glycol groups; it is more preferable that the macromonomer for forming a hydrogel has 4 or more polyalkylene glycol groups that have polymerization degrees of from 50 to 5,000, and it is still more preferable that the polymerizable substituent has an ethylenic unsaturated bond.

A fifth aspect of the invention provides a spheroid-containing hydrogel laminated body including two or more of the spheroid-containing hydrogel, which are disposed one on another in layers.

A sixth aspect of the invention provides a method of producing a spheroid-containing hydrogel, the method including:
a step of inoculating cells onto a substrate including a base material and plural hydrophilic regions and hydrophobic regions that are formed on the base material;
a spheroid formation step of culturing the inoculated cells to form cultured-cell-derived spheroids in the hydrophobic regions on the substrate;
a hydrogel-composite formation step of disposing a hydrogel on a side of the substrate, on which the spheroids are formed, to form a hydrogel composite; and
a step of detaching the substrate from the hydrogel composite to obtain a spheroid-containing hydrogel.
The hydrogel-composite formation step preferably includes:
a step of disposing a photosensitive composition including a hydrophilic polymer compound on a side of the substrate on which the spheroids are formed, so as to contact the spheroids with the photosensitive composition; and
a step of curing the photosensitive composition.

Further, the photosensitive composition preferably includes a macromonomer for forming a hydrogel, wherein the macromonomer has a weight average molecular weight of 10,000 or more, has 4 or more polyalkylene glycol groups, each having a polymerizable substituent at a terminal thereof, and has a tetra- or higher-valent linking group that binds to the polyalkylene glycol groups; it is more preferable that the macromonomer for forming a hydrogel has 4 or more polyalkylene glycol groups that have polymerization degrees of from 50 to 5,000, and it is still more preferable that the polymerizable substituent has an ethylenic unsaturated bond.

It is preferable that the culturing of the cells in the step of culturing the inoculated cells is conducted for at least 5 days.
The hydrophilic regions on the base material preferably include a polymer derived from a branched polyalkylene glycol derivative having 3 or more polyalkylene glycol groups, each having a polymerizable substituent at a terminal thereof, and a tri- or higher-valent linking group that binds to the polyalkylene glycol groups.
The hydrophilic regions are preferably formed on a temperature-responsive layer.
The hydrophobic regions are also preferably formed in an array on the base material.

### Advantageous Effects of Invention

According to the present invention, a spheroid composite including plural spheroids having a uniform size and disposed on a porous base material and an efficient production method thereof, and a multilayer spheroid composite formed by the spheroid composites disposed one on another in layers, can be provided.
According to the present invention, a spheroid-containing hydrogel having excellent function-maintainability and having two or more spheroids contained therein which are disposed in such a manner that the two or more spheroids do not contact each other, and a laminated body thereof, and an efficient method of producing a hydrogel including plural spheroids having a uniform size, can be provided.

### BRIEF DESCRIPTION OF THE DRAWINGS

FIG. 1 is a view that shows the state of cartilage cell spheroids formed on a porous base material according to an example of the present invention.
FIG. 2 is a view that shows the result of MTT staining of cartilage cell spheroids formed on a porous base material according to an example of the invention.
FIG. 3 is a view that shows the state of osteoblast spheroids formed on a substrate according to an example of the invention.
FIG. 4 is a view that shows the state of an osteoblast spheroid-containing hydrogel at 8th day of culturing according to an example of the invention.
FIG. 5 is a view that shows the result of MTT staining of an osteoblast spheroid-containing hydrogel at 8th day of culturing according to an example of the invention.

### BEST MODE FOR CARRYING OUT THE INVENTION

### (Spheroid composite)

The spheroid composite of the present invention includes:
a substrate including:
   a cell-adhesive porous base material; and
   plural hydrophilic regions and hydrophobic regions that are disposed on the porous base material and are formed by curing a photosensitive composition, wherein the photosensitive composition includes a branched polyalkylene glycol derivative having:
      3 or more polyalkylene glycol groups, each having a polymerizable substituent at a terminal thereof; and
      a tri- or higher-valent linking group that binds to the polyalkylene glycol groups; and
spheroids formed in the hydrophobic regions on the substrate.
In the spheroid composite, spheroids having a uniform size are formed on the porous base material, and thus spheroid maintainability is further improved.

The spheroid composite according to the invention is preferably produced by the below-described method of producing a spheroid composite. This allows the size of the spheroids to be uniformized more efficiently, and a spheroid composite having more favorable maintainability is obtained.
The specifics on the spheroid composite according to the invention are described below together with the method of producing a spheroid composite.

The multilayer spheroid composite according to the invention is formed by disposing two or more of the above-described spheroid composite one on another in layers. Due to this configuration, a multilayer spheroid composite in which plural spheroids having a uniform size are three-dimensionally disposed can be obtained.

The method of disposing in layers is not particularly limited, and usual methods may be used as appropriate. The number of spheroid composites to be disposed in layers is not particularly limited either, and the number of spheroid composites to be disposed in layers may be selected, as appropriate, in accordance with the purpose.

The multilayer spheroid composite thus obtained, in which spheroids having a uniform size are three-dimensionally disposed, readily exhibit tissue-like behavior when used as a graft.

The method of producing a spheroid composite according to the invention includes:
inoculating cells onto a substrate including:
   a cell-adhesive porous base material; and
   plural hydrophilic regions and hydrophobic regions that are disposed on the porous base material and are formed by curing a photosensitive composition, wherein the photosensitive composition includes a branched polyalkylene glycol derivative having:
      3 or more polyalkylene glycol groups, each having a polymerizable substituent at a terminal thereof; and
      a tri- or higher-valent linking group that binds to the polyalkylene glycol groups; and
culturing the inoculated cells to form cultured-cell-derived spheroids.
Use of the substrate allows plural spheroids having a uniform size to be formed efficiently in the hydrophobic regions on the porous base material, and also allows a spheroid composite including a substrate and spheroids formed on the substrate to be produced efficiently.

In the spheroid composite produced by the production method according to the invention, since the spheroids are formed on the porous base material, substances required for the maintenance of the spheroids can be supplied and unnecessary materials can be removed through the porous base material, whereby the maintainability of the spheroids is further improved.
Further, even in a case in which a multilayer spheroid composite is formed by disposing two or more of the spheroid composite, the individual spheroids can be maintained in a more favorable state.

When cells are cultured by using the multilayer fine spheroid composite having the above-described features, the cells are cultured to efficiently form a three-dimensional structure, and it is possible to supply a fresh culture liquid to the inner constituent cells of the three-dimensional structure, and to remove cell metabolites (beneficial physiologically-active substances and waste materials). More specifically, since the base material in the invention has liquid permeability as described above, it is possible to supply, through the base material (cell adhesion domain layer) in particular, a culture liquid to culture cells on and around the base material. Therefore, even in a case in which the number of the cells is increased due to culturing for a long time, necrosis of the inner cells can be prevented, and cell metabolites (beneficial physiologically-active substances and harmful waste materials) from not only the inner cells but also the all constituent cells can be collected in a circulating culture liquid, over time. In other words, the base material in the invention has a function similar to that of a capillary blood vessel in a biological tissue.
The spheroid composite including such a substrate and multicellular aggregates (spheroids) derived from cultured cells formed on the substrate serves as an excellent model of a biological organ from both the configurative viewpoint and the viewpoint of exhibition of functions, and is a quite useful material for development of artificial organs, development of an evaluation system for efficacy or toxicity of novel drugs, selection of anticancer drugs on an individual level, evaluation of cancer metastatic ability, and the like, considering that metabolites produced over time can be collected and measured.
Further, the spheroid composite is also thought to be applicable as a graft for the treatment of a wound such as bum or bedsore.

The spheroid composite according to the invention may be configured as a culturing apparatus for efficiently performing the three-dimensional culturing of cells. The configuration thereof is not particularly limited as long as the spheroid composite according to the invention is employed. For example, the spheroid composite may be configured as a culturing apparatus including a means for controlling liquid supply of a culture liquid to the spheroid composite. Examples of the configuration of such a culturing apparatus include a configuration including a means for controlling liquid supply of a culture liquid, which uses a pipette-type body or a dropper-type body that is supported by a support to stand vertically, or is attached to a culturing vessel without using a support, and a configuration including a pump as the means for supplying a culture liquid.

The spheroid composite according to the invention can be used, for example, as a field from which vasa vasorum is induced toward peripheral regions. Delivery of nutrients via blood vessels is important for tissues or organs of living bodies. Therefore, for example, formation of blood vessels can be induced by providing an angiogenic factor (growth factor) such as a physiologically-active factor or a basic fibroblast growth factor (b-FGF), and combining therewith a scaffold material of which the nanostructure and microstructure are controlled so as to support induction of blood vessels, such that invasion of blood vessels into the inside of spatially arranged cell aggregates is induced.
This allows provision of an environment that is closer to a living body, and self-tissue formation by cells can be effected with higher efficiency.

In the substrate, the hydrophilic regions are regions on which a crosslinked material formed by curing of the photosensitive composition described below has been formed, and the hydrophobic regions are regions other than the hydrophilic regions, wherein the porous base material is exposed at the hydrophobic regions.
The substrate according to the invention is preferably a substrate that is compartmentalized to have the plural hydrophilic regions and plural hydrophobic regions. Further, the numbers, sizes and shapes of the hydrophilic regions and hydrophobic regions are not particularly limited, and may be suitably selected in accordance with the purpose. Specifics of the substrate according to the invention and the preparation method thereof are described below.

In the invention, with a view to forming spheroids having a uniform size, it is preferable that the plural hydrophobic regions has a uniform size and shape, and it is more preferable that the plural hydrophobic regions are arranged in an array.
Further, with a view to efficiently forming a large amount of homogeneous spheroids, the hydrophobic regions each has a diameter of preferably from 50 to 500 µm, more preferably from 100 to 300 µm, in a case in which each hydrophobic region is formed to have a circular shape.

The method of producing a spheroid composite according to the invention includes inoculating cells onto the substrate, and culturing the inoculated cells to form cultured-cell-derived spheroids in the hydrophobic regions on the substrate.
By inoculating cells on the substrate and culturing the cells, cultured-cell-derived spheroids can be formed in the hydrophobic regions on the substrate. Specifically, since the cells are disposed on only the hydrophobic regions on the substrate and the cells do not adhere to the hydrophilic regions, spheroids that correspond to the number, size and shape of the hydrophobic regions can be formed.

The type of the cells to be inoculated onto the substrate and the tissue from which the cells are derived in the invention are not particularly limited as long as the cells are adherent cells. Examples include cells immediately after being sampled from a living body, and oncogenic established cell systems. Cells related to the functional expression and pathology of a specific organ are preferable. More specific examples include liver parenchymal cells related to drug metabolism, pancreatic β cells related to control of blood sugar level, osteoblasts related to regeneration of bones, cartilage cells, neural stem cells related to neurotransmission, hair matrix cells related to hair growth, cancer cells, fibroblasts, embryonic stem cells which can be induced to be differentiated into various cells, and mesenchymal stem cells. Further, nonparenchymal cells that interact with these cells are also usable.

Commonly-used culture media may be used for culturing various cells. The culture medium used for culturing may be any culture medium that is usually used for culturing animal cells, and examples thereof include various basic culture liquids (standard culture liquids) that contain no serum such as Iscove's medium, RPMI medium, Dulbecco's modified Eagle's medium (DMEM), MEM medium and F12 medium.
A serum for accelerating cell growth may be added to the culture medium, or, for example, a cell growth factor such as FGF, EGF or PDGF or a known serum component such as transferin may be added, as an alternate of the serum, to the culture medium. In the case of the serum addition, the concentration of the serum may be varied, as appropriate, in accordance with the culture state at the time of the addition, and may usually be from 5 vol% to 10 vol%. Various vitamins and antibiotics (AB) such as streptomycin may be added to the culture medium, as appropriate.

The culture conditions for various cells may be selected, as appropriate, in accordance with the cells. For example, culturing in an incubator at a temperature of 37°C under a CO₂ concentration of 5% may be applied as the culture conditions.
Further, for formation of spheroids derived from various cells, spheroids can be formed by culturing under usual culture conditions for from 2 hours to 2 days.

The method of inoculating various cells onto the substrate is not particularly limited, and usual methods may be employed, as appropriate. The density of embryonic stem cells to be inoculated is not particularly limited as long as spheroids can be formed, and may be selected, as appropriate, in accordance with the size of the substrate, the number and size of the hydrophobic regions, and the like. For example, the density may be adjusted to be from 1 × 10⁴ to 1 × 10⁸ cells/mL, and preferably adjusted to be from 1 × 10⁴ to 1 × 10⁶ cells/mL.

The time for which the cells are cultured on the substrate according to the invention is not particularly limited as long as the time is equal to or longer than the time during which the cells can form spheroids in the hydrophobic regions on the substrate. The time required for forming spheroids under usual culture conditions is, for example, from several tens of minutes to 48 hours.

Formation of spheroids derived from various cells in the invention can be confirmed based on morphology observation. Specifically, the inoculated cells gather in the hydrophobic regions as culture time elapses, and form cell aggregates (spheroids).

According to the method of producing a spheroid composite according to the invention, a spheroid composite including a large amount of spheroids having a uniform size and formed on a substrate can be obtained. Since the spheroids formed in a uniform size have substantially homogeneous properties, the spheroids can exhibit similar tendencies in various behaviors.

Feeder cells may be disposed, in advance, on the hydrophobic regions on the substrate according to the invention, if necessary. Specifically, in the invention, a feeder cell layer may be formed in the hydrophobic regions on the substrate according to the invention, and various cells may be cultured on the feeder cell layer formed.
Formation of the feeder cell layer in advance allows the formation of spheroids derived from various cells to proceed more efficiently, and the stability of the spheroids to be improved. Examples of the feeder cells include COS-1 cells, vascular endothelial cells (for example, "human umbilical vein endothelial cells" manufactured by Dainippon Pharma Co., Ltd).), and fibroblasts.

In the method of producing a spheroid composite according to the invention, the cell-adhesive porous base material is formed preferably on a temporary support, from the viewpoints of production efficiency and handling properties of the substrate.

Specifically, the method of producing a spheroid composite according to the invention preferably includes:
inoculating cells onto a substrate comprising:
   a cell-adhesive porous base material disposed on a temporary support; and
   plural hydrophilic regions and hydrophobic regions that are disposed on the porous base material and are formed by curing a photosensitive composition, wherein the photosensitive composition includes a branched polyalkylene glycol derivative having 3 or more polyalkylene glycol groups, each having a polymerizable substituent at a terminal thereof, and a tri- or higher-valent linking group that binds to the polyalkylene glycol groups;
culturing the inoculated cells to form cultured-cell-derived spheroids in the hydrophobic regions on the substrate, thereby forming a spheroid composite on the temporary support; and
separating the spheroid composite and the temporary support from each other.

The method of separating the spheroid composite and the temporary support from each other in the invention is not particularly limited, and usual methods may be used. For example, in the case of using a biodegradable porous base material as the porous base material, culturing for several days causes decomposition of a part of the biodegradable porous base material, as a result of which the spheroid composite and the temporary support can be separated from each other. A part of the biodegradable porous base material is decomposed also by setting the culture conditions to be weakly acidic or weakly basic, as a result of which the spheroid composite and the temporary support can be separated from each other.

Examples of the material of the temporary support include glass, thermoplastic resins, thermosetting resins, silicones, diamond, metals and ceramics. In the invention, the material is preferably glass or a thermoplastic resin, and more preferably glass, from the viewpoint of adhesiveness of the porous base material.

In the invention, the porous base material is preferably disposed on a temperature-responsive layer, characteristics of which change in a temperature-responsive manner, and it is more preferable that the temperature-responsive layer is disposed on the temporary support.
Provision of the cell-adhesive porous base material on the temperature-responsive layer allows the porous base material to be more easily separated from the temperature-responsive layer disposed on the temporary support.

The temperature-responsive layer may be configured to include a temperature-responsive polymer that exhibits hydrophobicity at its lower critical solution temperature or higher, and that exhibits hydrophilicity at the lower critical solution temperature or lower. In the invention, the temperature-responsive polymer is preferably a temperature-responsive polymer that exhibits hydrophobicity at a cell cultivation temperature (usually 37°C), and that exhibits hydrophilicity at a temperature condition at which the spheroid composite is separated from the temporary support is preferable.
Although the lower critical solution temperature is not particularly limited, the lower critical solution temperature is preferably a temperature that is lower than the cell cultivation temperature, from the viewpoint of separation of the porous base material from the temporary support.

The temperature-responsive polymer that can be suitably used in the invention has a lower critical solution temperature (T) of preferably from 0 to 80°C, and more preferably from 0 to 50°C, from the viewpoint of toxicity to the cultured cells.
Specific examples of the temperature-responsive polymer include poly-N-isopropylacrylamide (T=32°C), poly-N-n-propylacrylamide (T=21°C), poly-N-n-propylmethacrylamide (T=32°C), poly-N-ethoxyethylacrylamide (T=about 35°C), poly-N-tetrahydrofurfurylacrylamide (T=about 28°C), poly-N-tetrahydrofurfurylmethacrylamide (T=about 35°C) and poly-N,N-diethylacrylamide (T=32°C).

The temperature-responsive layer may include other polymers. Specific examples thereof include poly-N-ethylacrylamide, poly-N-isopropylmethacrylamide, poly-N-cyclopropylacrylamide, poly-N-cyclopropylmethacrylamide, poly-N-acryloylpyrrolidine, poly-N-acryloylpiperidine, poly(methyl vinyl ether), alkyl-substituted cellulose derivatives such as methylcellulose, ethylcellulose and hydroxypropylcellulose, polyalkylene oxide block copolymers such as a block copolymer of polypolypropylene oxide and polyethylene oxide and polyalkylene oxide block copolymers.

These temperature-responsive polymers are prepared by homopolymerization or copolymerization of monomers, homopolymers of which have lower critical solution temperatures of from 0 to 80°C. Examples of the monomer include (meth)acrylamide compounds, N-(or N,N-di)alkyl-substituted (meth)acrylamide derivatives, N,N-dialkyl-substituted (meth)acrylamide derivatives, (meth)acrylamide derivatives having a cyclic group and vinylether derivatives.
Monomers other than those described above may further be added and copolymerized in a case in which the lower critical solution temperature needs to be adjusted in accordance with the type of cells to be cultured, or in a case in which the interaction between the temperature-responsive layer and the temporary support needs to be increased, or in a case in which the hydrophilicity-hydrophobicity balance of the temperature-responsive layer needs to be adjusted, or the like. It is also possible to use a graft or block copolymer of at least one of the above polymer for use in the invention and at least one other polymer, or a mixture of at least one of the polymer according to the invention and at least one other polymer. The polymer may be crosslinked as long as the intrinsic properties of the polymer are not impaired.

The method of disposing the temperature-responsive layer on the temporary support in the invention is not particularly limited. For example, in a case in which glass is used as the temporary support, the temperature-responsive layer containing a temperature-responsive polymer can be formed on the glass substrate by surface-treating a glass substrate with a silane coupling agent, thereafter forming a photosensitive composition layer containing a monomer(s) for forming the temperature-responsive polymer on the glass substrate, and polymerizing the monomer(s) by, for example, irradiation with light.
Examples of the method of surface-treating with a silane coupling agent include a method of surface-treating using DATES ((N,N'-diethylamino)dithiocarbamoylpropyl(triethoxy)silane).
The method of polymerizing the monomer(s) may be usual radical polymerization, RAFT polymerization (Reversible Addition-Fragmentation Chain Transfer Polymerization) or the like.

In the invention, the substrate provided with the temperature-responsive layer may further be subjected to surface treatment with the cell adhesion protein described above. This enables more efficient formation of spheroids.

The substrate according to the invention includes a cell-adhesive porous base material, and plural hydrophilic regions and hydrophobic regions that are disposed on the porous base material and are formed by curing a photosensitive composition, wherein the photosensitive composition includes at least one kind of branched polyalkylene glycol derivative having 3 or more polyalkylene glycol groups, each having a polymerizable substituent at a terminal thereof, and a tri- or higher-valent linking group that binds to the polyalkylene glycol groups.

Known materials that have adhesiveness to the cells and permeability to substances required for maintaining the cells may be used, without particular limitation, as the porous base material. Further, the porous base material may have any shape/configuration, such as gel, fiber or nonwoven fabric.
For example, in regard to a method of producing a porous base material containing a gel substance, the porous base material can be produced by preparing a solution of a cell-adhesive polymer compound, and applying the solution to the temporary support by employing a commonly-used liquid film formation method such as coating, dipping or spin coating. The porous base material can alternatively be produced by a method of polymerizing a polymerizable biodegradable monomer on the temporary support.

An example of a method of producing a porous base material containing a fibrous substance is an electrospinning (ELSP) method. An ELSP method is a technique by which fibers having a diameter at a submicron scale can be easily produced. Specifically, in an ELSP method, a high voltage is applied to a solution of a cell-adhesive polymer compound (for example, polyglycolic acid) so as to spray the polymer compound solution onto an electrically conductive material such as an aluminum foil, and to form fibers formed by the polymer compound. Here, the diameter of the fiber can be suitably changed by adjusting the voltage to be applied, the concentration of the polymer compound solution, the fly distance of the spray and the like. Further, a three-dimensionally-structured thin film having a three-dimensional network can be formed by continuously forming fibers on the electrically conductive material. With this method it is possible to increase the film thickness of the thin film formed by the fibers, and it is also possible to produce a nonwoven fabric having a submicron mesh. The thus-produced nonwoven fabric may be retained on or fixed onto the temporary support (for example, glass, a resin or the like).

Further, in the invention, the porous base material may be formed of a woven material. In the invention, the "fiber" encompasses a thread-like material formed by a single fiber, a thread-like material formed by a bundle of the fibers, and the like. The woven material in the invention is a material obtained by weaving such a thread-like material. When the woven material is formed by a mesh material or nanofibers, the cells can be cultured more efficiently in a three-dimensional manner.
Further, the thread-like fibers may be an appropriate combination of fibers selected from fibers having a diameter of, for example, from several tens to several hundreds of micrometers, and other fibers. In the thread-like fibers or woven materials, plural types of fiber/woven material, and/or plural fibers/woven materials having different physical shapes and/or properties such as thread diameter or aperture mesh size of woven material may be used, as appropriate. In any case, it is preferable that the material forming the porous base material is capable of forming a spatial form for three-dimensional cultivation. Therefore, in the case of a mesh material, the mesh material retains the spatial shape in advance. For example, a mesh material having a mesh aperture of from about 10 to 1,000 µm, or the like is favorably used.

In the invention, the cell-adhesive compound for forming the porous base material is not particularly limited as long as the compound can be formed into a gelatinous or fibrous form, which allows adhesion of the cells and can be used as a scaffold.
Specifically, the porous base material preferably includes at least one of a biodegradable compound or an extracellular matrix from the viewpoint of the biocompatibility of the spheroid composite, and is more preferably at least one selected from polyglycolic acid, polylactic acid, poly(ε-caprolactone), gelatin, collagen, alginic acid, fibrin, an adhesion protein derived from lectin, polylysine or an adhesive oligopeptide.

Although the thickness of the porous base material is not particularly limited in the invention, the thickness may be, for example, from 0.5 µm to 5,000 µm, and is preferably from 1 µm to 500 µm from the viewpoints of biodegradability and biocompatibility.
From the viewpoints of spheroid formation properties and handling properties of the substrate, the porous base material in the invention is preferably a gelatinous material formed from at least one selected from polyglycolic acid, polylactic acid, poly(ε-caprolactone), gelatin, collagen, alginic acid, fibrin, an adhesion protein derived from lectin, polylysine or an adhesive oligopeptide, and provided, at a film thickness of from 1 µm to 500 µm, on the temporary support.

The plural hydrophilic regions disposed on the porous base material are regions that are formed by curing a photosensitive composition containing the below-described branched polyalkylene glycol derivative, and the hydrophobic regions are regions at which the porous base material is exposed.

### (Branched polyalkylene glycol derivative)

The branched polyalkylene glycol derivative according to the invention characteristically includes 3 or more polyalkylene glycol groups, each having a polymerizable substituent at a terminal thereof, and a tri- or higher-valent linking group that binds to the polyalkylene glycol groups.
The branched polyalkylene glycol derivative having such a configuration is capable of forming a hydrophilic crosslinked material. The hydrophilic crosslinked material has excellent temporal stability of non-adhesiveness to the cells. For example, with a substrate having hydrophilic regions and hydrophobic regions that are formed on a porous base material at high accuracy by using the branched polyalkylene glycol derivative according to the invention, cell aggregates that are arranged in a compartmentalized manner at high accuracy can be formed by culturing cells on the substrate since the cells specifically adhere to the hydrophobic regions only. Further, the hydrophilic regions have excellent temporal stability of non-adhesiveness to the cells, and thus the cell aggregates (spheroids) arranged in a compartmentalized manner can be maintained for a long time.

The number of polyalkylene glycol groups each having a polymerizable substituent at a terminal thereof in the branched polyalkylene glycol derivative according to the invention is 3 or more. When the number of the polyalkylene glycol groups included is 2 or less, the hydrophilic regions formed thereby has only insufficient stability of the non-adhesiveness to cells over time, and the cell aggregates arranged in a compartmentalized manner cannot be maintained for a long time in some cases.
The number of the polyalkylene glycol groups included is preferably 4 or more, more preferably from 4 to 64, and further preferably from 4 to 16, from the viewpoints of stability over time and favorable spheroid formation properties.

The polyalkylene glycol group in the invention is not particularly limited as long as the polyalkylene glycol group has a polymerizable substituent at a terminal thereof.
The polymerizable substituent is not particularly limited as long as it is a substituent having a polymerizable functional group and capable of binding to the terminal of the polyalkylene glycol. The manner of the binding of the polymerizable substituent to the terminal of the polyalkylene glycol may be a binding manner in which the polymerizable substituent is bonded via an oxygen atom derived from the polyalkylene glycol, or a binding manner in which the terminal hydroxyl group of the polyalkylene glycol is replaced by another element.

The above-described polymerizable substituent may be a polymerizable functional group itself, or a substituent that contains a polymerizable functional group and a linking group.
As the polymerizable functional group in the invention, commonly-used polymerizable functional groups may be used without limitation, examples of which include a group having an ethylenic unsaturated bond, and an azide group. In the invention, the polymerizable functional group is preferably at least one selected from a group having an ethylenic unsaturated bond or an azide group, and more preferably an azide group, from the viewpoint of pattern formation properties of the hydrophilic regions.

The linking group in the polymerizable substituent is not particularly limited as long as it is a group capable of linking the polymerizable functional group and the polyalkylene glycol group, and may be configured to include, for example, at least one selected from an alkylene group, an arylene group, a heteroarylene group, a carbonyl group, an oxygen atom, a nitrogen atom, a sulfur atom, a disulfide or a hydrogen atom.
Specific examples thereof include a carbonyl group, an arylene group, an alkylenecarbonyl group, a carbonylarylene group and a carbamoylarylene group.
The valency of the linking group may be di- or higher-valent. The linking group may be a tri- or higher-valent linking group that links the polyalkylene glycol and two or more polymerizable functional groups.

The polyalkylene glycol group in the polyalkylene glycol group having a polymerizable substituent at a terminal thereof is not particularly limited as long as it is a polyalkylene glycol group capable of imparting hydrophilicity to the branched polyalkylene glycol derivative according to the invention. For example, a polyalkylene glycol group containing an alkyleneoxy structural unit having from 2 to 4 carbon atoms (e.g., ethyleneoxy, n-propyleneoxy, isopropyleneoxy, butyleneoxy, isobutyleneoxy or the like) can be favorably used.

The alkyleneoxy structural units in the polyalkylene glycol group may include only one kind of alkyleneoxy structural unit, or may be a combination of two or more kinds of alkyleneoxy structural unit. When the polyalkylene glycol group is composed of a combination of two or more kinds of alkyleneoxy structural unit, the polyalkylene glycol group may be a block polymer or a random polymer.
From the viewpoint of hydrophilicity, the polymerization degree of the polyalkylene glycol group may be 5 or more, and a polyalkylene glycol group having a polymerization degree of from 5 to 1,000 can preferably be used; the polymerization degree is more preferably from 10 to 500.

The tri- or higher-valent linking group that binds to the polyalkylene glycol groups each having a polymerizable substituent at a terminal thereof in the invention is not particularly limited as long as the tri- or higher-valent linking group binds to the terminals of the at least 3 polyalkylene glycol groups to which the polymerizable substituents are not bound, and is capable of linking the polyalkylene glycol groups to each other. The bonding manner may be any of a covalent bond, a coordination bond or an ionic bond.
Specific examples of the linking group include a linking group derived from a saccharide, a linking group derived from a polyhydric alcohol, a linking group derived from a polyvalent carboxylic acid, and a metal atom capable of binding the polyalkylene-glycol-group-containing groups via coordination bonds.

Examples of the saccharide include glyceraldehyde, erythrose, ribose, and glucose. Examples of the polyhydric alcohol include glycerin, pentaerythritol, xylitol and sorbitol. Examples of the polyvalent carboxylic acid include propanetricarboxylic acid, citric acid and benzenetricarboxylic acid. Examples of the metal atom include gold, silver, platinum, nickel and copper.

In the invention, from the viewpoints of hydrophilicity and stability over time, the linking group is preferably a linking groups derived from a polyhydric alcohol, more preferably a linking group derived from glycerin or a linking group derived from pentaerythritol, and particularly preferably a linking group derived from a compound selected from glycerin, polyglycerin, pentaerythritol or polypentaerythritol.

The polyalkylene glycol group having a polymerizable substituent at a terminal thereof in the invention is preferably a substituted polyalkylene glycol group represented by the following General Formula (1) from the viewpoints of stability over time and favorable spheroid formation properties.

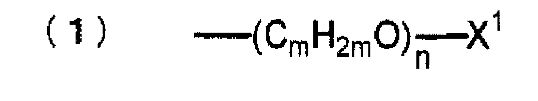

In General Formula (1), m represents an integer of from 2 to 4, preferably 2 or 3, and more preferably 2. Further, n represents an integer of from 5 to 1,000, preferably from 10 to 500, and more preferably from 10 to 300.

In General Formula (1), X¹ represents a polymerizable substituent. In the invention, the polymerizable substituent is preferably a polymerizable substituent represented at least one of the following General Formulas (3) and (4) from the viewpoint of the crosslink-curability of the branched polyalkylene glycol derivative according to the invention.

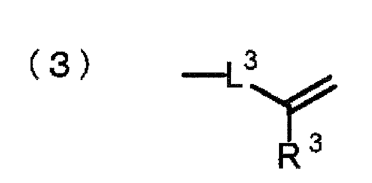

(4) -L⁴-N₃

In General Formula (3), L³ represents a single bond or a divalent linking group. The divalent linking group is not particularly limited as long as the divalent linking group is capable of linking the ethylenic unsaturated group and the polyalkylene glycol group. The divalent linking group may be, for example, a divalent linking group configured to include at least one of an alkylene group, an arylene group, a heteroarylene group, a carbonyl group, an oxygen atom, a nitrogen atom, an imino group or a hydrogen atom. The divalent linking group is preferably a divalent linking group selected from a carbonyl group, an ester group, an amide group, a phenylene group or an alkylene group having from 2 to 4 carbon atoms, or a divalent linking group formed by a combination thereof.
In the invention, L³ is more preferably a single bond, or a divalent linking group selected from a carbonyl group, a carbonylphenylene group or a carbamoylphenylene group.

R³ represents a hydrogen atom or an alkyl group having from 1 to 3 carbon atoms. Specific examples of the alkyl group having from 1 to 3 carbon atoms include a methyl group, an ethyl group, an n-propyl group and an isopropyl group. In the invention, R³ is preferably a hydrogen atom or a methyl group, and more preferably a hydrogen atom, from the viewpoint of crosslink-reactivity of the branched polyalkylene glycol derivative.

In General Formula (4), L⁴ represents a divalent linking group, and the definition and preferable ranges thereof are the same as in the case of the divalent linking group represented by L³.

In the invention, it is preferable that at least one of the polymerizable substituents is a substituent represented by the following General Formula (5). This configuration enables the reaction of the polymerizable substituent to be initiated by irradiation of light having a longer wavelength.

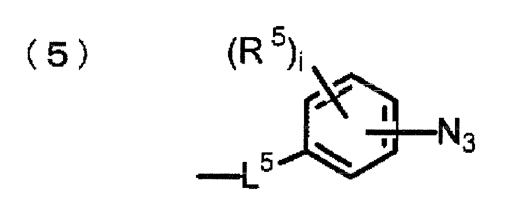

In General Formula (5), L⁵ represents a single bond or a divalent linking group. The specifics of the divalent linking group represented by L⁵ are the same as those of the divalent linking group represented by L³.

### Further, i represents 1 or 2.

In General Formula (5), R⁵ represents a substituent, and the substituent is not particularly limited as long as the substituent is capable of changing the absorption maximum wavelength of the polymerizable substituent represented by General Formula (5). In particular, the substituent is preferably a substituent capable of shifting the absorption maximum wavelength of the polymerizable substituent represented by General Formula (5) toward the longer wavelength side. Specific preferable examples of the substituent include a nitro group, a hydroxyl group, an alkyloxy group, a dialkylamino group, a cyano group and a nitroso group.

### When i is 2, two R⁵s may be the same or different.

The branched polyalkylene glycol derivative according to the invention is preferably a compound represented by the following General Formula (2) from the viewpoints of hydrophilicity and crosslink-reactivity.

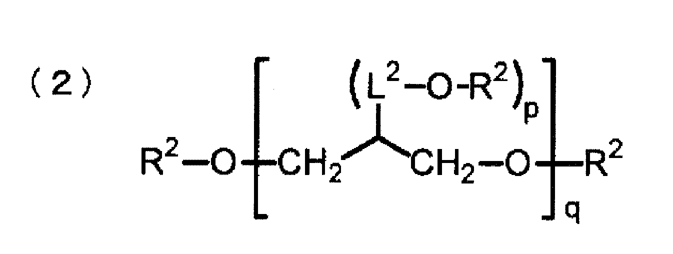

In General Formula (2), L² represents a single bond or a methylene group, and p represents 1 or 2. When p is 1, L² is preferably a single bond, and when p is 2, L² is preferably a methylene group.
q represents an integer of from 1 to 70. In the invention, when p is 1, q is preferably from 1 to 64, and more preferably from 2 to 10, from the viewpoints of hydrophilicity and crosslink-reactivity. When p is 2, q is preferably from 1 to 32, and more preferably from 1 to 5.

In General Formula (2), R² represents a polyalkylene glycol group having a polymerizable substituent at a terminal thereof or a polyalkylene glycol group having a hydroxyl group at a terminal thereof. In particular, R² is preferably a polyalkylene glycol group having a polymerizable substituent at a terminal thereof, and more preferably a substituted polyalkylene glycol group represented by General Formula (1), from the viewpoint of crosslink-reactivity.

From the viewpoints of stability over time and spheroid formation properties, it is preferable that the branched polyalkylene glycol derivative according to the invention has from 4 to 16 polyalkylene glycol groups each having a polymerizable substituent at a terminal thereof, and that the polyalkylene glycol groups are represented by General Formula (1), and that the polymerizable substituent is represented by at least one of General Formulas (3), (4) and (5).

The specific examples of the branched polyalkylene glycol derivative according to the invention are shown below, but the invention is not limited to these examples. The polymerization degree (n) of the polyalkylene glycol in the following specific examples means an average polymerization degree calculated from the weight average molecular weight of the branched polyalkylene glycol derivative. Further, the weight average molecular weight of the branched polyalkylene glycol derivative can be measured by, for example, gel permeation chromatography (GPC).

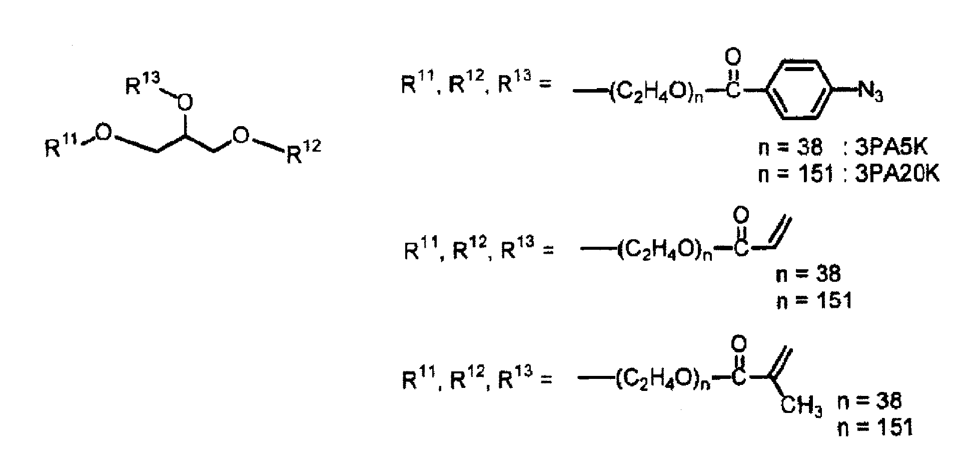

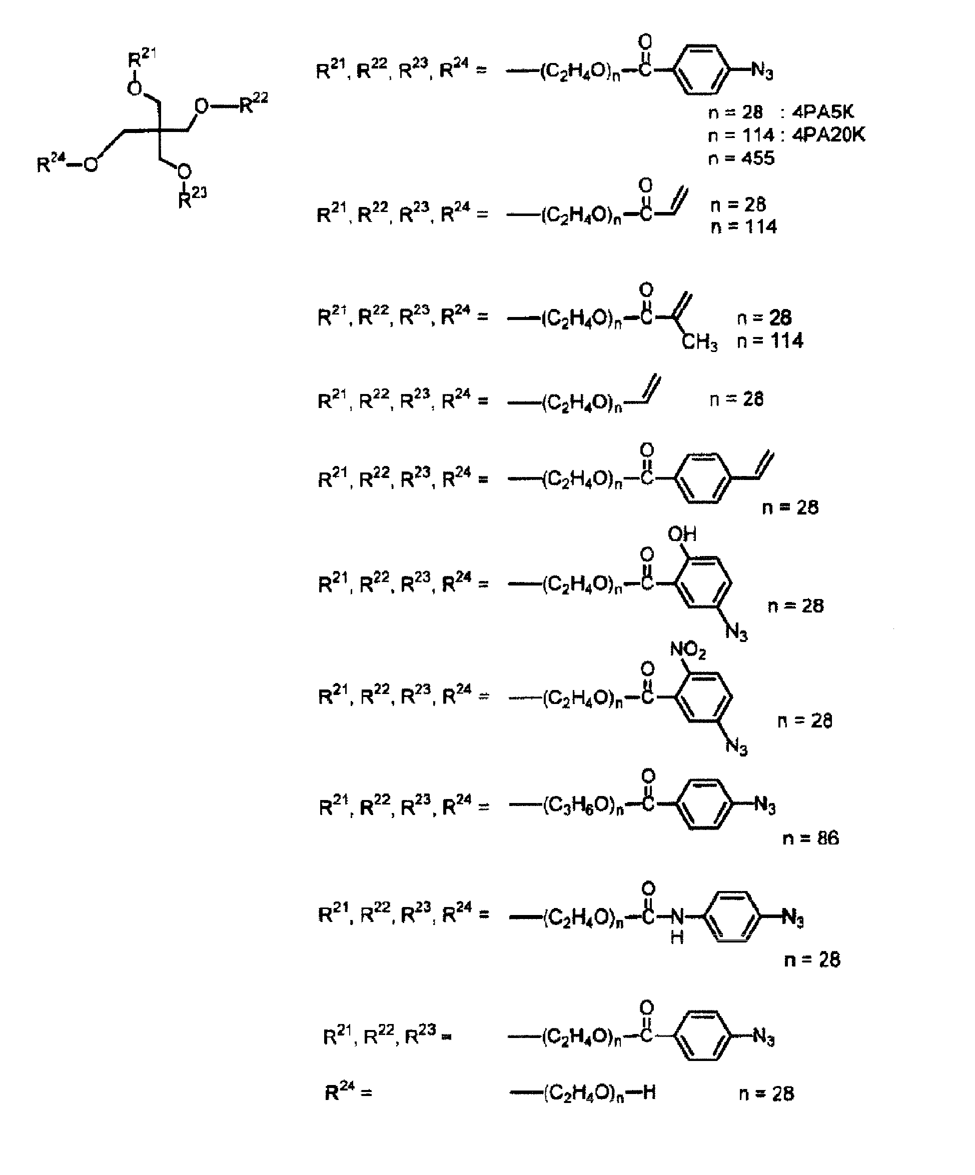

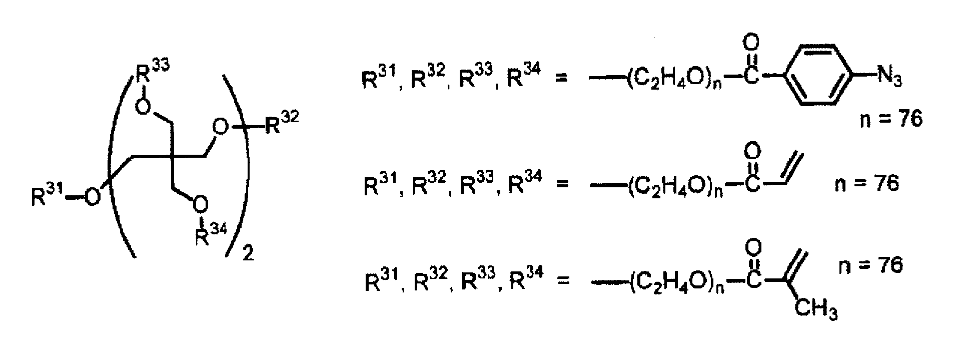

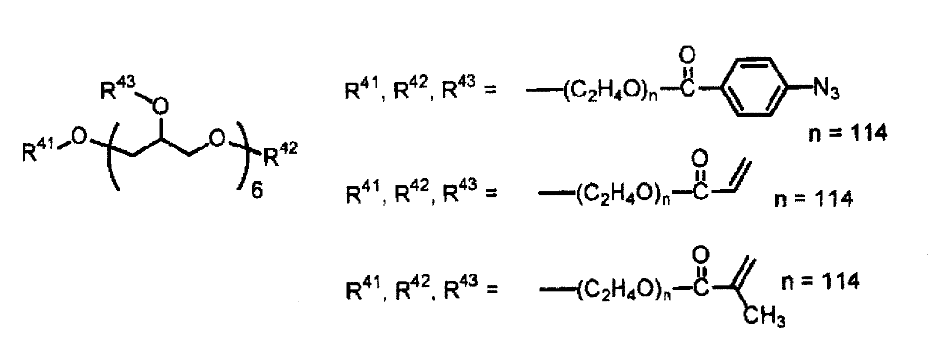

The branched polyalkylene glycol derivative according to the invention can be synthesized by, for example, binding polymerizable substituents to the terminal hydroxyl groups of a compound having three or more polyethylene glycol groups (hereinafter sometimes referred to as "multi-arm PEG", examples of which include SUNBRIGHT (registered trademark) PTE series, HGEO series and the like manufactured by NOF Corporation) via ester bonds, ether bonds or the like, using a commonly-employed method. For example, ester bonds can be formed by an acid-chloride method, an active ester method or the like.

The branched polyalkylene glycol derivative according to the invention may be used, for example, as a component of the photosensitive composition described below. Further, the branched polyalkylene glycol derivative according to the invention is capable of forming a hydrophilic crosslinked material through a crosslinking reaction.

The photosensitive composition according to the invention characteristically includes at least one kind of the branched polyalkylene glycol derivative. By using the photosensitive composition, for example, hydrophilic regions and hydrophobic regions, which are arranged in a compartmentalized manner at high accuracy, can be formed on the base material.
The photosensitive composition may include only one kind of the branched polyalkylene glycol derivative, or two or more kinds of the branched polyalkylene glycol derivative.
Further, the photosensitive composition may be configured to include various additives such as photopolymerization initiators, solvents, cell culture liquids, surfactants, buffer liquids, defoaming agents and antiseptic agents, in addition to the branched polyalkylene glycol derivative.

The photopolymerization initiator is not particularly limited as long as the photopolymerization initiator is capable of initiating a polymerization reaction when light is irradiated. The toxicity of the photopolymerization initiator against living cells is preferably low. Specific examples of the photopolymerization initiator include IRGACURE 2959 and IRGACURE 184 (both manufactured by Ciba Japan), of which IRGACURE 2959 is preferable from the viewpoints of cytotoxicity and water-solubility.

The solvent is not particularly limited as long as the solvent is capable of dissolving the branched polyalkylene glycol derivative. The "capable of dissolving" as used herein refers to capability of dissolving the branched polyalkylene glycol derivative in an amount of 0.1 % by mass or more.
Specific preferable examples of the solvent include: organic solvents such as benzene, toluene, THF, DMF and chloroform; and water. The solvent may be used by singly, or in mixture of two or more thereof.

The content ratio of the branched polyalkylene glycol derivative in the photosensitive composition may be, for example, from 0.1 to 50% by mass, and is preferably from 0.1 to 20% by mass.

The crosslinked material in the invention is formed by crosslink-curing the photosensitive composition. The crosslink-curing is not particularly limited as long as it is caused by polymerization reaction resulting from irradiation with light, and conditions for crosslink-curing may be suitably selected in accordance with the photosensitive composition.

The method of producing the substrate according to the invention may include, for example, a step of applying a photosensitive composition containing the branched polyalkylene glycol derivative to a porous base material so as to form a photosensitive composition layer, and a curing step of subjecting the photosensitive composition layer to light exposure treatment. As a result of these steps, a substrate including the crosslinked material formed on the porous base material can be produced.
If necessary, the method of producing the substrate may further include a heating step, a washing step, a drying step, a sterilization step and the like, after the curing step.

Usual thin film formation methods may be employed, without particular limitations, for the step of forming the photosensitive composition layer on the porous base material in the invention is not particularly limited. For example, a coating method, a dip coating method, a spin coating method or the like can favorably be applied.
The layer thickness of the photosensitive composition layer formed on the porous base material is not particularly limited, and may be suitably selected in accordance with the purpose of use of the substrate. The layer thickness of the photosensitive composition layer may be, for example, from 5 nm to 1,000 µm. In particular, the layer thickness is preferably from 10 nm to 1,000 nm, more preferably from 10 nm to 500 nm, from the viewpoints of spheroid formation properties.

The step of forming the photosensitive composition layer on the porous base material may include a step of removing solvent in the photosensitive composition, if necessary. The conditions for the step of removing solvent may be suitably selected in accordance with the solvent, and may be drying at ordinary temperature or drying by heating. The drying may be performed, for example, at from 30 to 150°C for from 1 minute to 10 hours, and is preferably performed at from 35 to 120°C for from 3 minutes to 1 hour.

The light exposure treatment in the curing step may be a step of exposing the entire face of the photosensitive composition layer to light, or a step of conducting partial exposure of the photosensitive composition layer to light in a desired pattern. In the invention, the step of conducting partial exposure to light in a desired pattern is preferable, and it is more preferable that a development step subsequent to the step of conducting partial exposure to light is included. As a result, a substrate in which hydrophilic regions formed by the crosslinked material and hydrophobic regions in which the crosslinked material is not formed are patternwise formed on a porous base material, can be produced.

The step of conducting partial exposure to light in a desired pattern is preferably a step of conducting partial exposure to light in a desired pattern via a mask (photomask) having light transmissivity. When the partial exposure to light is conducted using a mask tightly attached to the photosensitive composition layer, patternwise light exposure can be performed with higher accuracy.

The light source used for the light exposure is not particularly limited as long as it is a light source with which the photosensitive composition layer can be cured. Examples of the light source include X-ray, electron beam, excimer laser, a xenon lamp, a metal halide lamp, a low-pressure mercury lamp and a high-pressure mercury lamp. In particular, a low-pressure or high-pressure mercury lamp can favorably be used, and a high-pressure mercury lamp of from 10 to 2,000 W is preferable.
Further, the exposure wavelength and the exposure amount are not particularly limited either, and may be suitably selected in accordance with the photosensitive composition. The exposure wavelength may be, for example, from 200 to 400 nm, and is preferably from 280 to 400 nm. The exposure amount may be, for example, from 0.1 to 1,000 mJ/cm² and is preferably from 1 to 200 mJ/cm², more preferably from 10 to 20 mJ/cm²_{.}

The development step is not particularly limited as long as a method capable of removing regions of the photosensitive composition layer that have not been exposed to light from the porous base material is employed, and examples thereof include washing by using a solvent and immersion in a solvent. In the invention, washing by using water as a solvent and immersion in water are preferable.

When the substrate according to the invention is prepared to be a substrate in which the hydrophilic regions formed by the crosslinked material and the hydrophobic regions at which the porous base material is exposed are formed, in a compartmentalized (patterned) state, on a base material, the substrate can be more favorably used as a spheroid culture substrate. Specifically, cells are arranged in only the hydrophobic regions, and the cells do not adhere to the hydrophilic regions, as a result of which the spheroids can be arranged in a desired pattern.

### (Spheroid-containing Hydrogel)

The spheroid-containing hydrogel according to the invention is a spheroid-containing hydrogel that includes a hydrogel, and two or more spheroids having a uniform size with a diameter of from 70 µm to 400 µm and arranged in the hydrogel in such a manner that the two or more spheroids do not contact each other.
Since the spheroids are contained within the hydrogel and have a specific uniform size, a spheroid-containing hydrogel configured to have excellent and homogenous function-maintainability of spheroids can be realized.

In the invention, the spheroids contained within the hydrogel have a diameter of from 70 µm to 400 µm. The diameter of the spheroids is preferably from 70 µm to 300 µm, and more preferably from 100 µm to 200 µm, from the viewpoint of the function-maintainability of the spheroids. When the size of the spheroids is more than 400 µm, the spheroids become difficult to live on. When the size is less than 70 µm, the functions as the spheroids cannot be exerted.
In the invention, the sizes of the two or more spheroids included in the hydrogel are uniform. As used herein, the "are uniform" encompasses a case in which there is no significant difference between the sizes of the individual spheroids and the biological functionality of the spheroids is homogeneous, as well as a case in which the sizes are identical.

Further, the two or more spheroids contained within the hydrogel in the invention are disposed in such a manner that the two or more spheroids do not contact each other. The distance between two spheroids is not particularly limited, and is preferably 30 µm or more, more preferably from 30 µm to 200 µm, and further preferably from 50 µm to 150 µm, from the viewpoint of function-maintainability of the spheroids. As used herein, the distance between spheroids means the minimum distance between the outermost contours of two spheroids.
The specifics of the spheroid-containing hydrogel in the invention are described below together with a method of producing a spheroid-containing hydrogel.

The spheroid-containing hydrogel laminated body according to the invention includes two or more of the spheroid-containing hydrogel described above, which are disposed one on another in layers. Due to this configuration, a spheroid-containing hydrogel laminated body in which plural spheroids having a uniform size are three-dimensionally arranged can be obtained.

The method of disposing in layers is not particularly limited, and usual methods may be used, as appropriate. For example, the disposing in layers may be conducted such that sides from which the substrates have been detached contact each other, or the disposing in layers may be conducted such that a side from which the substrate has been detached and a side opposite thereto contact each other. Further, the number of spheroid-containing hydrogels to be disposed in layers is not particularly limited either, and the number of spheroid-containing hydrogels to be disposed in layers may be suitably selected in accordance with the purpose.

In the thus-obtained spheroid-containing hydrogel laminated body, spheroids having a uniform size are three-dimensionally disposed. Therefore, when the spheroid-containing hydrogel laminated body is used as, for example, a graft, the hydrogel laminated body tends to exhibit a tissue-like behavior.

The method of producing a spheroid-containing hydrogel according to the invention includes:
a step of inoculating cells onto a substrate including a base material and plural hydrophilic regions and hydrophobic regions that are formed on the base material;
a step of culturing the inoculated cells to form cultured-cell-derived spheroids in the hydrophobic regions on the substrate;
a hydrogel-composite formation step of disposing a hydrogel on a side of the substrate, on which the spheroids are formed, to form a hydrogel composite; and
a step of detaching the substrate from the hydrogel composite to obtain a spheroid-containing hydrogel.
Since the method has such steps, a hydrogel including plural spheroids having a uniform size can be produced efficiently.

Further, the hydrogel including spheroids is a material into which not only oxygen but also various substances permeate and diffuse easily, and the hydrogel is a soft material. Therefore, even in a case in which plural cell aggregates (spheroids) are formed in the hydrogel, it is possible to maintain the individual spheroids efficiently without causing physical damages to cells. Disposing the hydrogels, each of which include plural spheroids, one on another in layers enables the spheroids in the three-dimensionally arranged state to be maintained and cultured.
In the below, the method of producing a spheroid-containing hydrogel according to the invention is described in detail.

The method of producing a spheroid-containing hydrogel according to the invention includes a step of inoculating cells onto a substrate including a base material and plural hydrophilic regions and hydrophobic regions that are formed on the base material.
Commonly-used base materials may be used, without particular limitation, as the base material of the substrate. Examples of the material of the base material include glass, thermoplastic resins, thermosetting resins, silicones, diamond, metals and ceramics. In the invention, the material is preferably glass or a thermoplastic resin, and more preferably glass, from the viewpoint of adhesiveness between the base material and the crosslinked material.

The base material in the invention is preferably subjected to surface treatment with at least one selected from a silane coupling agent having an amino group, a silane coupling agent having an ethylenic unsaturated group or a polylysine. The surface treatment allows improvement in the stability of binding between the base material and the crosslinked material formed thereon.

Further, it is also preferable that the base material is a base material that has been subjected to surface treatment with at least one cell adhesion protein, and is more preferably a base material that has been subjected to surface treatment with at least one selected from a silane coupling agent having an amino group, a silane coupling agent having an ethylenic unsaturated group or a polylysine and further subjected to surface treatment with at least one cell adhesion protein.
By using a base material surface-treated with a cell adhesion protein for forming the substrate, cell aggregates can be formed more efficiently, for example, in a case in which cells are cultured on the substrate.
Examples of the cell adhesion protein include collagen, gelatin, fibronectin, vitronectin, laminin, teinecin and elastin. Of these, collagen, gelatin, fibronectin and vitronectin are preferable, and collagen and gelatin are more preferable, from the viewpoint of cell aggregate formation properties.

In the invention, the base material preferably has a temperature-responsive layer thereon. The temperature-responsive layer allows more efficient spheroid transfer from the substrate to the hydrogel.
The temperature-responsive layer may be configured to contain a temperature-responsive polymer that exhibits hydrophobicity at a lower critical solution temperature or higher, and that exhibits hydrophilicity at the lower critical solution temperature or lower. In the invention, the temperature-responsive polymer is preferably a temperature-responsive polymer that exhibits hydrophobicity at a cell cultivation temperature (usually 37°C), and that exhibits hydrophilicity at a temperature condition at which the substrate is detached from the hydrogel composite.
The lower critical solution temperature is not particularly limited, and is preferably a temperature lower than the cell cultivation temperature from the viewpoint of spheroid transfer properties.

Examples of this temperature-responsive polymer are the same as the above examples of the temperature-responsive polymer in the spheroid composite, and preferable embodiments thereof are also the same.
Examples of the method of disposing the temperature-responsive layer on the base material are the same as the above examples of the method of disposing the temperature-responsive layer on the temporary support in the spheroid composite, and preferable embodiments thereof are also the same.

As the method of forming plural hydrophilic regions and hydrophobic regions on the base material, commonly-used methods may be employed without particular limitation. Specific examples thereof include a method using plasma etching and a method using photolithography.

The method using plasma etching may be, for example, a method including:
forming a hydrophilic composition layer containing a hydrophilic polymer on the base material;
removing at least a part of the hydrophilic composition layer by, for example, plasma etching using a metal mask, thereby forming hydrophobic regions in which the base material is exposed, as a result of which hydrophilic regions in which the hydrophilic composition layer remains and hydrophobic regions are formed on the base material.

The method using photolithography may be, for example, a method including forming a photosensitive composition layer containing a hydrophilic polymer on the base material, and forming, on the base material, hydrophilic regions formed by curing the photosensitive composition layer and hydrophobic regions formed by removing uncured photosensitive composition layer, using a usual photolithography technique.

Polymer compounds containing a hydrophilic group may be used, without particular limitation, as the hydrophilic polymer contained in the hydrophilic composition layer or the photosensitive composition layer. The hydrophilic group may be a cationic group, an anionic group or a nonionic group.
The hydrophilic polymer in the invention is preferably a polymer containing a nonionic group as a hydrophilic group, and more preferably a polymer containing a polyalkylene glycol group, from the viewpoint of spheroid formation properties.

The polymer containing a polyalkylene glycol group may be a polyalkylene glycol itself, or a branched polyalkylene glycol derivative having two or more polyalkylene glycol groups.
Examples of the branched polyalkylene glycol derivative include SUNBRIGHT (registered trademark) PTE series, HGEO series and the like manufactured by NOF Corporation.

In the invention, the method of forming plural hydrophilic regions and hydrophobic regions on the base material is preferably a method using photolithography, from the viewpoint of substrate production efficiency. The hydrophilic regions preferably include a polymer that is derived from a branched polyalkylene glycol derivative having three or more polyalkylene glycol groups, each having a polymerizable substituent at a terminal thereof, and a tri- or higher-valent linking group bonded to the polyalkylene glycol groups, from the viewpoint of spheroid formation properties on the substrate.

The branched polyalkylene glycol derivative according to the invention preferably has three or more polyalkylene glycol groups, each having a polymerizable substituent at a terminals thereof, and a tri- or higher-valent linking group that binds to the polyalkylene glycol groups.
The branched polyalkylene glycol derivative having such a configuration is capable of forming a hydrophilic crosslinked material. The non-adhesiveness of the hydrophilic crosslinked material to cells has excellent stability over time. For example, when cells are cultured on a substrate including hydrophilic regions and hydrophobic regions that are formed on the base material at high accuracy by using the branched polyalkylene glycol derivative according to the invention, cell aggregates that are arranged in a compartmentalized manner at high accuracy can be formed since the cells specifically adhere to only the hydrophobic regions. The hydrophilic regions have excellent stability with respect to non-adhesiveness to cells over time, and thus cell aggregates (spheroids) in which the cells have formed a three-dimensional aggregation state can be maintained for a long time. In addition, since the spheroids formed have sizes corresponding to the hydrophobic regions, spheroid aggregates having a uniform size can be formed easily.

Examples of the branched polyalkylene glycol derivative used for the production of the spheroid-containing hydrogel according to the invention are the same as the above examples of the branched polyalkylene glycol derivative in the spheroid composite, and preferable embodiments thereof are also the same.
The method of forming the hydrophilic regions on the base material by using the branched polyalkylene glycol derivative according to the invention may include, for example, forming a photosensitive composition layer on the base material by using a photosensitive composition containing the branched polyalkylene glycol derivative, and forming the hydrophilic regions by a crosslinking reaction.

The photosensitive composition according to the invention preferably includes at least one of the branched polyalkylene glycol derivative. Use of the photosensitive composition allows, for example, formation of hydrophilic regions and hydrophobic regions, which are arranged in a compartmentalized manner in a uniform size at high accuracy, on the base material.
The photosensitive composition may include only one of the branched polyalkylene glycol derivative, or two or more of the branched polyalkylene glycol derivative.
Further, the photosensitive composition may be configured to include, in addition to the branched polyalkylene glycol derivative, various additives such as photopolymerization initiators, solvents, cell culture liquids, surfactants, buffer liquids, defoaming agents and antiseptic agents.

The photopolymerization initiator is not particularly limited as long as the photopolymerization initiator is capable of initiating a polymerization reaction when light is irradiated. The toxicity of the photopolymerization initiator against living cells is preferably low. Specific examples of the photopolymerization initiator include IRGACURE 2959 and IRGACURE 184 (both manufactured by Ciba Japan), and IRGACURE 2959 is preferable from the viewpoints of cytotoxicity and water-solubility.

The solvent is not particularly limited as long as the solvent is capable of dissolving the branched polyalkylene glycol derivative. The "capable of dissolving" as used herein refers to capability of dissolving the branched polyalkylene glycol derivative in an amount of 0.1 % by mass or more.
Specifically, organic solvents such as benzene, toluene, THF, DMF and chloroform, and water are preferable for use as the solvent. Further, the solvents may be used singly, or in mixture of two or more thereof.

The content ratio of the branched polyalkylene glycol derivative in the photosensitive composition may be, for example, from 0.1 to 50% by mass, and is preferably from 0.1 to 20% by mass.

The method of producing the substrate according to the invention may include, for example, a step of applying a photosensitive composition containing the branched polyalkylene glycol derivative to a base material so as to form a photosensitive composition layer, and a curing step of subjecting the photosensitive composition layer to light exposure treatment. As a result of these steps, a substrate including the crosslinked material formed on the base material can be produced.
If necessary, the method of producing the substrate may further include a heating step, a washing step, a drying step, a sterilization step and the like, after the curing step.

The step of forming the photosensitive composition layer on the base material in the invention is not particularly limited, and usual thin film formation methods may be employed. For example, a coating method, a dip coating method, a spin coating method and the like can favorably be applied.
The layer thickness of the photosensitive composition layer formed on the base material is not particularly limited, and may be suitably selected in accordance with the purpose of use of the substrate. The layer thickness of the photosensitive composition layer may be, for example, from 5 nm to 1,000 µm. In the invention, the layer thickness of the photosensitive composition layer is preferably from 10 nm to 1,000 nm, and more preferably from 10 nm to 500 nm, from the viewpoints of spheroid formation properties and spheroid maintainability.

The step of forming the photosensitive composition layer on the base material may include a step of removing solvent in the photosensitive composition, if necessary. The conditions for the step of removing solvent may be suitably selected in accordance with the solvent, and may be drying at ordinary temperature or drying by heating. The drying may be performed, for example, at from 30 to 150°C for from 1 minute to 10 hours, and is preferably performed at from 35 to 120°C for from 3 minutes to 1 hour.

The light exposure treatment in the curing step may be a step of exposing the entire face of the photosensitive composition layer to light, or a step of conducting partial exposure of the photosensitive composition layer to light in a desired pattern. In the invention, the step of conducting partial exposure to light in a desired pattern is preferable, and it is more preferable that a development step subsequent to the step of conducting partial exposure to light is included. As a result, a substrate in which hydrophilic regions formed by the crosslinked material and hydrophobic regions in which the crosslinked material is not formed are patternwise formed on a base material, can be produced.

The step of conducting partial exposure to light in a desired pattern is preferably a step of conducting partial exposure to light in a desired pattern via a mask (photomask) having light transmissivity. When the partial exposure to light is conducted using a mask tightly attached to the photosensitive composition layer, patternwise light exposure can be performed with higher accuracy.

The light source used for the light exposure is not particularly limited as long as it is a light source with which the photosensitive composition layer can be cured. Examples of the light source include X-ray, electron beam, excimer laser, a xenon lamp, a metal halide lamp, a low-pressure mercury lamp and a high-pressure mercury lamp. In particular, a low-pressure or high-pressure mercury lamp can favorably be used, and a high-pressure mercury lamp of from 10 to 2,000 W is preferable.
Further, the exposure wavelength and the exposure amount are also not particularly limited either, and may be suitably selected in accordance with the photosensitive composition. The exposure wavelength may be, for example, from 200 to 400 nm, and is preferably from 280 to 400 nm. The exposure amount may be, for example, from 0.1 to 1,000 mJ/cm², and is preferably from 1 to 200 mJ/cm², more preferably from 10 to 20 mJ/cm².

The development step is not particularly limited as long as a method capable of removing regions of the photosensitive composition layer that have not been exposed to light from the base material is employed, and examples thereof include washing by using a solvent and immersion in a solvent. In the invention, washing by using water as a solvent and immersion in water are preferable.

The shapes of the hydrophilic regions and hydrophobic regions in the invention are not particularly limited, and may be suitably selected in accordance with the purpose. For example, a hydrophobic region may be formed in a circular shape, a polygonal shape such as a triangle shape, an oval shape or a stripe shape. The sizes of the hydrophilic regions and hydrophobic regions are not particularly limited either, and may be suitably selected in accordance with the purpose.

When a hydrophobic region formed on the substrate according to the invention is formed in a circular shape, the size thereof is preferably such that the diameter thereof is from 5 µm to 1,000 µm, and more preferably from 50 µm to 500 µm. The width of a hydrophilic region that separate adjacent hydrophobic regions is preferably from 50 µm to 500 µm, and more preferably from 100 µm to 200 µm. The thickness of the layer in the hydrophilic regions is preferably from 10 nm to 1,000 nm, and more preferably from 10 nm to 500 nm.
When the hydrophilic regions and hydrophobic regions have the sizes described above, highly-functional spheroids can be produced more efficiently, and can be maintained for a longer time.
The shapes and sizes of the hydrophilic regions and hydrophobic regions in the invention can easily be controlled at high accuracy by applying light exposure treatment via a mask as the light exposure treatment in the curing step.

The cells to be inoculated onto the substrate in the invention is not particularly limited in terms of the species thereof and the tissue from which the cells are derived, as long as the cells are adherent cells. Examples include cells immediately after being sampled from a living body, and oncogenic established cell systems. The cells are preferably related to the functional expression and pathology of a specific organ. More specific examples include liver parenchymal cells related to drug metabolism, pancreatic β cells related to control of blood sugar level, osteoblasts related to regeneration of bones, cartilage cells, neural stem cells related to neurotransmission, hair matrix cells related to hair growth, cancer cells, fibroblasts, embryonic stem cells that can be induced to differentiate into various cells, and mesenchymal stem cells. Nonparenchymal cells that interact with these cells are also usable.

The method of producing a spheroid-containing hydrogel according to the invention includes a step of culturing the cells inoculated onto the substrate to form cultured-cell-derived spheroids in the hydrophobic regions on the substrate.
When the substrate including plural hydrophilic regions and hydrophobic regions formed on the base material is used, the cells are arranged only in the hydrophobic regions, and the cells do not adhere to the hydrophilic regions. As a result, spheroids are formed in only the hydrophobic regions on the substrate.

As the method of culturing cells on the substrate in the invention, usual cell culture methods may be employed without limitation. For example, desired cells can be selectively arranged in the hydrophobic regions on the substrate by providing a cell culture medium on the substrate, inoculating desired cells onto the cell culture medium, and applying culture conditions that are selected in accordance with the desired cells.

Examples of usual cell culture media for various cells include Dulbecco's modified Eagle's culture medium (DMEM), MEMα and and RPMI 1640. The cell culture medium is suitably selected in accordance with the kind of cells to be cultured. Further, various additives applicable to usual cell culture, such as serum, various vitamins and various antibiotics, may be added to these culture media, as necessary. The concentration of these additives may be a commonly-employed concentration, and, for example, the concentration of serum may be from 5 to 10 vol% of the amount of the culture medium.
The culture conditions for various cells may be suitably selected in accordance with the cells. The conditions may be, for example, at 5% CO₂ and 37°C.
The concentration of the cells inoculated into the culture medium may be, for example, from 1 × 10⁴ to 1 × 10⁸ cells/mL, and preferably from 1 × 10⁴ to 1 × 10⁶ cells/mL.

In the invention, feeder cells may be provided in advance in the hydrophobic region, if necessary. Specifically, in the invention, a feeder cell layer may be formed in the hydrophobic regions on the substrate, and the cells may be cultured on the formed feeder cell layer.
Provision of the feeder cell layer in advance allows formation of the spheroids by the cultured cells to proceed more efficiently, and improves the stability of the spheroids. Examples of the feeder cells include COS-1 cells, vascular endothelial cells (for example, "human umbilical vein endothelial cells" manufactured by Dainippon Pharma Co., Ltd.) and fibroblasts.

The time for which the cells on the substrate are cultured in the invention is not particularly limited as long as it is equal to or longer than a time during which the cells can form spheroids in the hydrophobic regions on the substrate. The time required for the formation of spheroids is, when usual culture conditions are employed, from several tens of minutes to 48 hours, for example.
In the invention, it is preferable to continue cultivation of the cells even after formation of spheroids, from the viewpoint of spheroid retainability in the hydrogel. Therefore, cell culture is preferably conducted for at least 5 days after inoculation of the cells, and more preferably for from 7 to 30 days after inoculation of the cells.
As a result of culturing the cells for 5 days or longer, the spheroids become to exhibit functions similar to those exhibited in a living body, spheroid transfer property from the substrate to the hydrogel is improved, and spheroid retainability in the hydrogel is further improved.

The method of producing a spheroid-containing hydrogel according to the invention includes a hydrogel-composite formation step of disposing a hydrogel on a side of the substrate at which the spheroids have been formed, so as to form a hydrogel composite.
By disposing a hydrogel on a side of the substrate on which the spheroids have been formed such that the hydrogel and the spheroids contact each other, a hydrogel composite which includes the substrate, spheroids and hydrogel, and in which the spheroids on the substrate have been transferred to the hydrogel, is formed.

As the hydrogel, those obtained by making a hydrophilic polymer insoluble in water by, for example, crosslinking, and swelling the resultant polymer with water, may be used without particular limitation. Specific examples include: synthetic polymers such as polyoxyalkylene glycol and various polyoxyalkylene glycol derivatives; natural polymers such as gelatin, collagen, hyarulonic acid and polysaccharides (cellulose); polymers obtained by chemical modification of natural polymers; and any mixture of the above polymers.
Further, the method of disposing the hydrogel on the substrate is not particularly limited as long as the hydrogel and the spheroids on the substrate are allowed to contact each other, and commonly-used methods may be employed.

In the invention, the hydrogel-composite formation step preferably includes:
a step of disposing a photosensitive composition containing a hydrophilic polymer on a side of the substrate on which the spheroids are formed, so as to contact the spheroids with the photosensitive composition; and
a step of curing the photosensitive composition.
As a result, the hydrogel composite can be formed more efficiently.

As the hydrophilic polymer, hydrophilic polymers that can be made insoluble in water by crosslinking may be used without particular limitation. Examples thereof include: synthetic polymers such as polyoxyalkylene glycol and various polyoxyalkylene glycol derivatives; natural polymers such as gelatin and polysaccharides (cellulose); and polymers obtained by chemical modification of natural polymers.
The method of crosslinking the hydrophilic polymer may be suitably selected in accordance with the kind of hydrophilic polymer.

The hydrophilic polymer in the invention is preferably a macromonomer for forming a hydrogel, wherein the macromonomer has a weight average molecular weight of 10,000 or more, has 4 or more polyalkylene glycol groups, each having a polymerizable substituent at a terminal thereof, and has a tetra- or higher-valent linking group that binds to the polyalkylene glycol groups, from the viewpoints of hydrogel formation properties, the handling properties of the hydrogel and spheroid maintainability.

Examples of the macromonomer for forming a hydrogel in the invention (hereinafter sometimes referred to as "branched polyalkylene glycol derivative") are the same as the above examples of the branched polyalkylene glycol derivative in the spheroid composite, and preferable embodiments thereof are also the same. Specifically, the number of polyalkylene glycol groups each having a polymerizable substituent at a terminal thereof included is preferably 4 or larger, more preferably from 4 to 64, and further preferably from 8 to 16, from the viewpoints of retainability and maintainability of the spheroids.

The content ratio of the macromonomer for forming a hydrogel in the photosensitive composition may be, for example, from 0.1 to 50% by mass, and is preferably from 1 to 20% by mass.

In the invention, the photosensitive composition may be configured to include at least one hydrophilic polymer (preferably the macromonomer for forming a hydrogel formation), and optionally include other components, as necessary.
Examples of other components include various additives such as photopolymerization initiators, cell culture liquids, surfactants, buffer liquids, defoaming agents and antiseptic agents.

In the invention, the photosensitive composition preferably includes at least one of a cell culture liquid, a buffer liquid, physiological saline or water, from the viewpoints of hydrogel formation properties and spheroid retainability.

The photopolymerization initiator is not particularly limited as long as the photopolymerization initiator can initiate a polymerization reaction when light is irradiated. The toxicity of the photopolymerization initiator against living cells are preferably low. Specific examples of the photopolymerization initiator include IRGACURE 2959 and IRGACURE 184 (both manufactured by Ciba Japan), and IRGACURE 2959 is preferable from the viewpoints of cytotoxicity and water-solubility.

The method of disposing the photosensitive composition on the substrate in the invention is not particularly limited, and usual liquid application methods may be employed. For example, a coating method, a dripping method or the like may be favorably used.
The layer thickness of the photosensitive composition layer formed by the photosensitive composition applied onto the substrate is not particularly limited as long as the layer thickness is equal to or larger than a layer thickness that allows the spheroids on the substrate to be covered by the photosensitive composition. The layer thickness of the photosensitive composition layer may be suitably selected in accordance with the purpose, and may be, for example, from 50 µm to 3,000 µm. The layer thickness of the photosensitive composition layer is preferably from 50 µm to 1,000 µm, and more preferably from 50 µm to 300 µm.

The method of curing the photosensitive composition is not particularly limited as long as the photosensitive composition can be cured by the method, and is preferably light exposure treatment.
The light source used for the light exposure is preferably a light source with which the photosensitive composition layer can be cured, and of which toxicity to living cells is low. A low-pressure or high-pressure mercury lamp, for example, may favorably be used as the light source, and a high-pressure mercury lamp of from 10 to 2,000 W is preferable.
Further, the exposure wavelength and the exposure amount are not particularly limited as long as the toxicity of the resultant conditions against living cells is low, and the exposure wavelength and the exposure amount may be suitably selected in accordance with the photosensitive composition. The exposure wavelength may be, for example, from 200 to 400 nm, and preferably from 320 to 400 nm. The exposure amount may be, for example, from 1 to 200 mW/cm² preferably from 5 to 100 mW/cm², and more preferably from 10 to 50 mW/cm²_{.}

The method of producing a spheroid-containing hydrogel according to the invention includes a step of detaching the substrate from the hydrogel composite so as to obtain the spheroid-containing hydrogel. By detaching only the substrate after formation of the hydrogel composite, a spheroid-containing hydrogel can be produced more efficiently.

The method of detaching the substrate from the hydrogel composite is not particularly limited, and usual methods may be employed. For example, the hydrogel may be grasped with tweezers, and detached from the substrate. Since the spheroids formed on the substrate have been transferred from the substrate to the hydrogel, simple detachment of the hydrogel results in recovery of the spheroids in the hydrogel.
In a case in which the temperature-responsive polymer is disposed in the hydrophobic regions on the substrate, the spheroids can be efficiently detached from the substrate and transferred to the hydrogel by decreasing the temperature after forming the hydrogel on the spheroid substrate. As a result of detachment of the hydrogel in this manner, the spheroids are recovered in the hydrogel with higher efficiency.
The time between the formation of the hydrogel composite and the detachment of the substrate is not particularly limited, and is preferably within 72 hours, more preferably within 24 hours, from immediately after formation of the composite, from the viewpoint of the spheroid retainability.

The disclosures of Japanese Patent Application Nos. 2008-230223 and 2008-230224 are incorporated herein by reference in.their entirety.
All documents, patent applications, and technical standards mentioned in this specification are herein incorporated by reference to the same extent as if each individual document, patent application, or technical standard was specifically and individually indicated to be incorporated by reference.

### EXAMPLES

Hereinafter, the invention is described more specifically with reference to examples. However, the invention is not limited by these examples. Unless otherwise indicated, "%" represents "% by mass".

First, a branched polyalkylene glycol derivative used in the invention, and a substrate produced using the photosensitive composition containing the polyalkylene glycol are described specifically.

### (Reference Example 1)

### - Synthesis of branched polyalkylene glycol derivative (4PA20K) -

12 g (93.6 mmol) of 4-azide-benzoic acid was dissolved in 40 mL of thionyl chloride, and the mixture was heated under reflux for 1.5 hours. The reaction mixture was concentrated under reduced pressure, and a small amount of hexane was added thereto. The mixture was concentrated again under reduced pressure, and dried under vacuum, as a result of which 9.3 g (51.2 mmol, yield 70%) of 4-azide-benzoic acid chloride, which is a target product, as a white solid was obtained.
¹H-NMR (CDCl₃) δ: 8.11-8.15 (2H, m), 7.11-7.16 (2H, m).

Next, 81 mg (0.8 mmol) of triethylamine and 147 mg (1.2 mol) of 4-dimethylaminopyridine were added to 3 mL of dichloromethane (dehydrated), and the mixture was stirred while cooling in an ice bath. To this solution, a solution (5 mL) of 363 mg (2.0 mmol, 5 molar equivalents relative to the terminal OH groups of the multi-arm PEG) of the above-obtained 4-azide-benzoic acid chloride in dichloromethane (dehydrated) was dropwise added, and the mixture, as is, was continued to be stirred for 5 minutes. Thereafter, the reaction container was shielded from light, and a solution (20 mL) of 2 g (0.1 mmol) of a multi-arm PEG (SUNBRIGHT (registered trademark) PTE-20000 manufactured by NOF Corporation, a pentaerythritol derivative having 4 polyethylene glycol groups) in dichloromethane (dehydrated) was dropwise added slowly. The reaction solution was taken out from the ice bath, and the reaction solution, as is, was stirred at room temperature for 18 hours. The reaction mixture was concentrated under reduced pressure, and suspended by adding benzene. The suspension was filtered to remove salt, and thereafter concentrated again under reduced pressure. The processes of dissolving the crude product in a small amount of benzene, dropwise adding the resultant solution to isopropyl ether cooled at 0°C, and recovering the obtained precipitate by filtration, were repeated three times, and the white solid obtained was dried under reduced pressure, as a result of which 1.74 g (yield 85%) of a desired branched polyalkylene glycol derivative (4PA20K) was obtained.
The ratio of replacement of the terminal hydroxyl groups by polymerizable substituents calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

Branched polyalkylene glycol derivatives were synthesized in the same manner as in Reference Example 1, except that the multi-arm PEGs shown in the following Table 1 were used as the multi-arm PEG, instead of PTE-20000. The yields, characteristics and the like are shown in Table 1.

**Table 1**

| Branched polyalkylene glycol derivative | Multi-arm PEG | Average molecular weight of multi-arm PEG | Number of PEG groups | Ratio of substitution (%) | Yield (%) | Characteristics |
|---|---|---|---|---|---|---|
| 4PA5K | SUNBRIGHT PTE-5000 | 5000 | 4 | 100 | 80 | White solid |
| 4PA20K | SUNBRIGHT PTE-20000 | 20000 | 4 | 85 | 85 | White solid |
| 8PA20K | SUNBRIGHT HGEO-20000 | 20000 | 8 | 92 | 93 | White solid |
| 8PA40K | SUNBRIGHT HGEO-40000 | 40000 | 8 | 84 | 85 | White solid |

### (Reference Example 2)

15.3 g (0.1 mol) of 5-amino-salicylic acid was suspended in a mixed solution of 80 mL of distilled water and 20 mL of concentrated hydrochloric acid, and the suspension was stirred at room temperature for 30 minutes. The mixed solution was cooled in an ice bath, and 10 mL aqueous solution of 6.9 g (0.1 mol) of sodium nitrite was dropwise added at a velocity at which the liquid temperature of the reaction liquid of the solution did not exceed 5°C, and stirring was continued for 1 hour. Then, 30 mL aqueous solution of 7.15 g (0.11 mol) of sodium azide was dropwise added at a velocity at which the liquid temperature of the reaction liquid did not exceed 10°C. The ice bath was removed, and, while the temperature of the mixture was allowed to return to room temperature, the mixture was stirred vigorously until generation of air bubbles stopped. The precipitate generated was collected by filtration, and, further, the precipitate was washed with distilled water. The solid obtained was air-dried in the dark, and completely dried under reduced pressure, as a result of which 12.0 g (67.0 mmol, yield=67%) of 5-azide-salicylic acid as a white solid was obtained.
¹H-NMR (DMSO-d₆) δ: 11.14 (1H, bs), 7.41 (1H, d, J = 3.0 Hz), 6.88 (1H, dd, J = 8.5, 3.0 Hz), 6.68 (1H, d, J = 8.4 Hz).

5 g (27.9 mmol) of the obtained 5-azide-salicylic acid was suspended in 50 mL of thionyl chloride, and the suspension was stirred at 70°C for 1 hour. The reaction mixture was allowed to cool to room temperature, and excess thionyl chloride was removed under reduced pressure, as a result of which a red solid of 5-azide-salicylic acid chloride was obtained quantitatively.
¹H-NMR (DMSO-d₆) δ: 7.39 (1H, d, J = 2.9 Hz), 7.26 (1H, dd, J = 8.8,2.9 Hz), 7.00 (1H, d,J=8.8Hz).

81 mg (0.8 mmol) of triethylamine and 147 mg (1.2 mol) of dimethylaminopyridine were then added to 3 mL of dichloromethane (dehydrated), and the mixture was stirred while cooling in an ice bath. To this solution, a solution (5 mL) of 358 mg (2 mmol, 5 molar equivalents relative to the terminal OH groups of the multi-arm PEG) of the above-obtained 5-azide-salicylic acid chloride in dichloromethane (dehydrated) was dropwise added, and the mixture, as is, was continued to be stirred for 5 minutes. Thereafter, the reaction container was shielded from light, and a solution (20 mL) of 2 g (0.1 mmol) of a multi-arm PEG (SUNBRIGHT (registered trademark) PTE-20000 manufactured by NOF Corporation, a compound having 4 polyethylene glycol groups) in dichloromethane (dehydrated) was dropwise added slowly. The reaction solution was taken out from the ice bath, and the reaction solution, as is, was stirred at room temperature for 18 hours. The reaction mixture was concentrated under reduced pressure, and suspended by adding benzene. The suspension was filtered to remove salt, and thereafter concentrated again under reduced pressure. The processes of dissolving the crude product in a small amount of benzene, dropwise adding the resultant solution to isopropyl ether cooled at 0°C, and recovering the obtained precipitate by filtration were repeated three times, and the white solid obtained was dried under reduced pressure, as a result of which 1.77 g (yield 85%) of a desired branched polyalkylene glycol derivative (4PB20K) was obtained.
The ratio of replacement of the terminal hydroxyl groups by polymerizable substituents calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 3)

1.72 g (yield 85%) of a desired branched polyalkylene glycol derivative (8PB20K) was obtained in the same manner as in Example 4, except that HGEO-20000 (manufactured by NOF Corporation, a hexaglycerin derivative having eight polyethylene glycol groups) was used as the multi-arm PEG instead of PTE-20000 in Reference Example 2.
The ratio of replacement of the terminal hydroxyl groups by polymerizable substituents calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 4)

18.1 g (0.1 mol) of 5-amino-2-nitrobenzoic acid was suspended in a mixed solution of 80 mL of distilled water and 20 mL of concentrated hydrochloric acid, and the suspension was stirred at room temperature for 30 minutes. The mixed solution was cooled in an ice bath, and 10 mL aqueous solution of 6.9 g (0.1 mol) of sodium nitrite was dropwise added at a velocity at which the liquid temperature of the reaction liquid of the solution did not exceed 5°C, and the mixture, as is, was stirred for 1 hour. Then, 30 mL aqueous solution of 7.15 g (0.11 mol) of sodium azide was dropwise added at a velocity at which the liquid temperature of the reaction liquid did not exceed 10°C. The ice bath was removed, and, while the temperature of the mixture was allowed to return to room temperature, the mixture was stirred vigorously until generation of air bubbles stopped. The generated precipitate was collected by filtration, and, further, the precipitate was washed with distilled water. The solid obtained was air-dried in the dark and completely dried under reduced pressure, as a result of which 19.2 g (92.4 mmol, yield=92%) of 5-azide-2-nitrobenzoic acid as a white solid was obtained.
¹H-NMR (DMSO-d₆) δ:13.99 (1H, bs), 8.09-8.06 (1H, m), 7.45-7.42 (2H, m).

1.0 g (4.8 mmol) of the obtained 5-azide-2-nitrobenzoic acid was suspended in 10 mL of thionyl chloride, and the suspension was stirred at 70°C for 1 hour. The reaction mixture was allowed to cool to room temperature, and excess thionyl chloride was removed under reduced pressure, as a result of which a red solid of 5-azide-salicylic acid chloride was obtained quantitatively.
¹H-NMR (DMSO-d₆) δ: 8.10-8.07 (1H, m), 7.46-7.42 (2H, m).

81 mg (0.8 mmol) of triethylamine and 147 mg (1.2 mol) of dimethylaminopyridine were then added to 3 mL of dichloromethane (dehydrated), and the mixture was stirred while cooling in an ice bath. To this solution, a solution (5 mL) of 417 mg (2 mmol, 5 molar equivalents relative to the terminal OH groups of the multi-arm PEG) of the above-obtained 5-azide-2-nitrobenzoic acid chloride in dichloromethane (dehydrated) was dropwise added, and stirring was continued for 5 minutes. The reaction container was shielded from light, and a solution (20 mL) of 2 g (0.1 mmol) of a multi-arm PEG (SUNBRIGHT (registered trademark) PTE-20000 manufactured by NOF Corporation, a compound having 4 polyethylene glycol groups) in dichloromethane (dehydrated) was dropwise added slowly. The reaction solution was taken out from the ice bath, and the reaction solution, as is, was stirred at room temperature for 18 hours. The reaction mixture was concentrated under reduced pressure, and suspended by adding benzene. The suspension was filtered to remove salt, and thereafter concentrated again under reduced pressure. The processes of dissolving the crude product in a small amount of benzene, dropwise adding the resultant solution to isopropyl ether cooled at 0°C, and recovering the obtained precipitate by filtration were repeated three times, and the white solid obtained was dried under reduced pressure, as a result of which 1.81 (yield 85%) of a desired branched polyalkylene glycol derivative (4PC20K) was obtained.
The ratio of replacement of the terminal hydroxyl groups by polymerizable substituents calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 5)

1.71 g (yield 85%) of a desired branched polyalkylene glycol derivative (8PC20K) was obtained in the same manner as in Reference Example 4, except that HGEO-20000 (manufactured by NOF Corporation, a hexaglycerin derivative having eight polyethylene glycol groups) was used as the multi-arm PEG instead of PTE-20000 in Reference Example 4.
The ratio of replacement of the terminal hydroxyl groups by polymerizable substituents as calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 6)

A solution (5 mL) of 181 mg (2.0 mmol, 5 molar equivalents relative to the terminal OH groups of the multi-arm PEG) of acryloyl chloride in benzene (dehydrated) was dropwise added, and the mixture, as is, was continued to be stirred for 5 minutes. Thereafter, the reaction container was shielded from light, and a solution (20 mL) of 2 g (0.1 mmol) of a multi-arm PEG (SUNBRIGHT (registered trademark) PTE-20000 manufactured by NOF Corporation, a pentaerythritol derivative having 4 polyethylene glycol groups) in benzene (dehydrated) was dropwise added slowly. The reaction solution was taken out from the ice bath, and the reaction solution, as is, was stirred at room temperature for 18 hours. The reaction mixture was concentrated under reduced pressure, and suspended by adding benzene. The suspension was filtered to remove salt, and thereafter concentrated again under reduced pressure. The processes of dissolving the crude product in a small amount of benzene, dropwise adding the resultant solution to isopropyl ether cooled at 0°C, and recovering the obtained precipitate by filtration were repeated three times, and the white solid obtained was dried under reduced pressure, as a result of which 1.74 g (yield 85%) of a desired branched polyalkylene glycol derivative (4PD20K) was obtained.
The ratio of replacement of the terminal hydroxyl groups by polymerizable substituents as calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 7)

1.74 g (yield 85%) of a desired branched polyalkylene glycol derivative (8PD20K) was obtained in the same manner as in Reference Example 6, except that HGEO-20000 (manufactured by NOF Corporation, a hexaglycerin derivative having eight polyethylene glycol groups) was used as the multi-arm PEG instead of PTE-20000 in Reference Example 6.
The ratio of replacement of the terminal hydroxyl groups by polymerizable substituents as calculated from an integral ratio of ¹H-NMR was 85%.
¹H-NMR (CDCl₃) δ: 8.05 (8H, d, J = 8.6 Hz), 7.07 (8H, d, J = 8.6 Hz), 4.46 (8H, t, J = 4.9 Hz), 3.89-3.39 (2145H, m).

### (Reference Example 8)

A desired branched polyalkylene glycol derivative (8PD40K) was obtained in the same manner as in Reference Example 6, except that HGEO-40000 (manufactured by NOF Corporation, a hexaglycerin derivative having eight polyethylene glycol groups) was used as the multi-arm PEG instead of PTE-20000 in Reference Example 6.

### (Example 1)

A white cut glass slide (manufactured by Matsunami Glass Ind., Ltd., circular type having a diameter of 21 mm) serving as a temporary support was immersed in a 0.1 % aqueous solution of poly-L-lysine (PLL) for 2 hours, and dried to form a thin film made of PLL on the glass slide. This was then immersed in a 0.15% aqueous solution of gelatin for 2 hours, and dried to form a porous base material made of PLL and gelatin on the glass slide.

The branched polyalkylene glycol derivative (4PA20K) prepared in Reference Example 1 was dissolved in toluene to prepare a toluene solution of 4PA20K (1%) as a photosensitive composition A.
As a porous base material, the thin film made of PLL and gelatin disposed on the temporary support obtained above was used. 110 µL of the photosensitive composition A was dripped on the thin film, and a film was formed using a spin coating method (500 rpm × 5 seconds+3,000 rpm × 20 seconds+6,000 rpm × 1 second), and dried by being left to stand at ordinary temperature. The film was brought into close contact with a photomask made of quartz glass (on which a number of circular patterns, each having a diameter of 200 µm, are arranged), and exposed to light using a high-pressure mercury lamp (200 W) for 40 seconds. Thereafter, the film was washed with deionized water (development step: running water for 15 seconds+immersion for 20 minutes). The film was dried at ordinary temperature, as a result of which a substrate having a microfabricated hydrophilic crosslinked material on its surface and formed on the temporary support was obtained. Observation of the substrate surface using a phase-difference optical microscope (magnification × 100) confirmed that a fine micropattern was formed at high accuracy.

The substrate formed on the temporary support was subjected to sterilization treatment, and set on the bottom face of a 12-well plate manufactured by FALCON. DMEM (containing 10 vol% of FBS as serum; the culture media used in the following contained serum in the same manner) was added thereto as a culture medium, and bovine articular cartilage cells (chondrocyte P-3) were inoculated at a cell concentration of 5 × 10⁶ cells/mL (2 mL/well). The cells were cultured under culture conditions of 5% CO₂ and 37°C, whereby cartilage cell aggregates (spheroids), which had a uniform size and were arranged in a pattern corresponding to the hydrophobic regions formed on the base material, were obtained within 24 hours.
The substrate on which the spheroids were formed was separated from the temporary support by culturing for several days (from 1 day to 21 days), as a result of which a spheroid composite was obtained.

Further, a spheroid composite in which cartilage cell aggregates (spheroids) having a uniform size were formed was obtained in a case when a photosensitive composition prepared using the branched polyalkylene glycol derivative prepared in any of Reference Examples 2 to 5 instead of 4PA20K was used, as well as in a case in which a photosensitive composition prepared using the polyalkylene glycol derivative prepared in any of Reference Examples 6 and 7 and having a concentration of IRGACURE 2959 (photopolymerization initiator) of 0.05% was used.

### (Example 2)

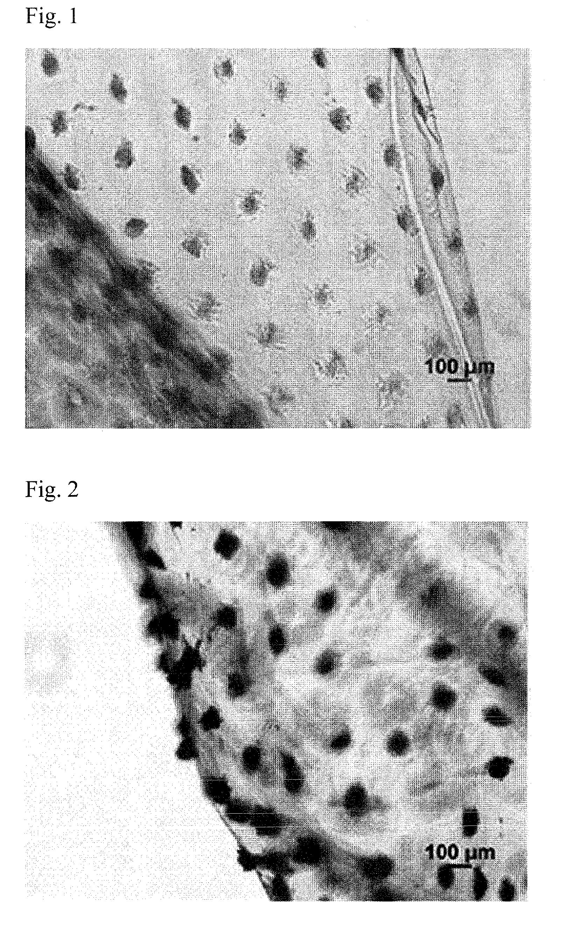

A spheroid composite was obtained in the same manner as in Example 1, except that a thin film of PLA formed by dripping 200 µl of a toluene solution (concentration: 10 mg/ml) of polylactic acid (PLA, Mn: 20 K) on a glass slide, thereafter forming a film by a spin coating method (2,000 rpm × 30 seconds) and drying the film by leaving the film to stand at ordinary temperature, was used as the porous base material formed on the temporary support instead of the thin film made of PLL and gelatin in Example 1.
The state of the spheroid composite separated from the temporary support is shown in FIG. 1. Further, MTT staining of the spheroid composite separated from the temporary support is shown in FIG. 2.

### (Example 3)

A spheroid composite was obtained in the same manner as in Example 2, except that polycaprolactone (PCL, Mn: 42.5 K, Mw: 65 K) was used instead of PLA in Example 2.

### (Example 4)

A spheroid composite was obtained in the same manner as in Example 2, except that a thin film in which PLL was coated on the PLA thin film, and which was prepared by forming the thin film of PLA on the glass slide, and thereafter immersing the resultant in a 0.1 % aqueous solution of PLL for 2 hours followed by drying, was used as the porous base material formed on the temporary support, instead of the thin film made of PLA in Example 2.

### (Example 5)

A spheroid composite was obtained in the same manner as in Example 4, except that PCL was used instead of PLA in Example 4.

### (Example 6)

A spheroid composite was obtained in the same manner as in Example 4, except that a 0.15% aqueous solution of gelatin was used instead of the 0.1% aqueous solution of PLL in Example 4.

### (Example 7)

A spheroid composite was obtained in the same manner as in Example 5, except that a 0.15% aqueous solution of gelatin was used instead of the 0.1 % aqueous solution of PLL in Example 5.

### (Example 8)

A white cut glass slide (manufactured by Matsunami Glass Ind., Ltd., circular type having a diameter of 21 mm) was washed with ozone (15 minutes × 2 times). This was immersed in a mixed solution of 1 ml of a coupling agent DATES ((N,N'-diethylamino)dithiocarbamoyl propyl(triethoxy)silane), 8 ml of chloroform, 1 ml of methanol and 85 µl of concentrated hydrochloric acid for 30 minutes. Thereafter the glass slide was dried at 70°C for 30 minutes, washed sequentially with chloroform, methanol and Milli-Q water, and dried in a desiccator under reduced pressure.
The resultant was put in a solid Teflon (registered trademark) container, and the container was filled with a 4 mol/l solution of IPAAm (isopropylacrylamide) in THF that had been bubbled with argon gas for 1 hour. A quartz glass was placed on the glass slide in such a manner that air is not incorporated, and the glass slide was irradiated with UV at 25 W/cm² for 10 minutes. Thereafter the glass slide was washed sequentially with methanol and Milli-Q water, and dried, as a result of which a temporary support having a temperature-responsive layer was obtained.

The temporary support having a temperature-responsive layer obtained above was used. 200 µl of a toluene solution of PLA (polylactic acid, Mn: 20 K) having a concentration of 10 mg/ml was dripped on the temperature-responsive layer of the temporary support, and a thin film was formed using a spin coating method (2,000 rpm × 30 seconds), and dried.
The thin film was then immersed in 1.5 µl/ml of fibronectin at 37°C for 2 hours.
Through the above processes, a porous base material made of PLA and fibronectin was formed on the temperature-responsive layer disposed on the temporary support.

A substrate having plural hydrophilic regions and hydrophobic regions formed on the temporary support was produced in the same manner as in Example 1, except that the above-obtained glass slide having the porous base material made of PLA and fibronectin formed on the temperature-responsive layer was used instead of the glass slide on which a porous base material made of PLL and gelatin was formed in Example 1.
Cells were cultured to form a spheroid composite on the temporary support in the same manner as in Example 1, except that the thus-produced substrate was used.
Then, the spheroid composite could be separated from the temporary support by putting the composite formed on the temporary support, together with the temporary support, under a temperature condition of 25°C.

### (Example 9)

A spheroid composite was produced in the same manner as in Example 8, except that PCL (polycaprolactone, Mn: 42.5 K, Mw: 65 K) was used instead of PLA in Example 8.

### (Example 10)

The substrate produced in the same manner as in Example 1 was subjected to sterilization treatment, and set on the bottom face of a 12-well plate manufactured by FALCON. Using DMEM (containing 10 vol% of FBS as serum) as a culture medium, bovine aorta endothelial cells (BAEC) were inoculated at a cell concentration of 5 × 10⁶ cells/mL (2 mL/well). The cells were cultured under culture conditions of 5% CO₂ and 37°C, as a result of which the cells arranged within 24 hours in a pattern corresponding to the hydrophobic regions formed on the base material. Rat primary hepatic cells were inoculated, at a cell concentration of 5 × 10⁶ cells/mL (2 mL/well), onto the substrate on which BAECs had been cultured in pattern, using WILLIAMS' MEDIUM E (containing 10 vol% of FBS as serum) as a culture medium. The cells were cultured under culture conditions of 5% CO₂ and 37°C, and hepatic cell aggregates (spheroids) having a uniform size were obtained on the patterned BAECs within 24 hours.
The substrate on which the spheroids were formed was separated from the temporary support by culturing for several days (from 1 day to 21 days), as a result of which a spheroid composite was obtained.
Further, spheroid composites were respectively obtained in the same manner as above by using substrates, which were produced in the same manner as in Examples 2 to 9, instead of the substrate produced in Example 1.

### (Example 11)

The substrate produced in the same manner as in Example 1 was subjected to sterilization treatment, and set on the bottom face of a 12-well plate manufactured by FALCON. Using DMEM (containing 10 vol% of FBS as serum; the culture media used in the following contained serum in the same manner) as a culture medium, murine fibroblasts (NIH-3T3) were inoculated at a cell concentration of 5 × 14⁶ cells/mL (2 mL/well). The cells were cultured under culture conditions of 5% CO₂ and 37°C, as a result of which the cells arranged in a pattern corresponding to the hydrophobic regions formed on the base material within 24 hours. Using WILLIAMS' MEDIUM E (containing 10 vol% of FBS as serum) as a culture medium, rat primary hepatic cells were inoculated, at a cell concentration of 5 × 10⁶ cells/mL (2 mL/well), onto the substrate on which NIH-3T3 cells had been cultured in pattern. The cells were cultured under culture conditions of 5% CO₂ and 37°C, as a result of which hepatic cell aggregates (spheroids) having a uniform size were obtained on the patterned NIH-3T3 cells within 24 hours.
The substrate on which the spheroids were formed was separated from the temporary support by culturing for several days (from 1 day to 21 days), as a result of which a spheroid composite was obtained.
Further, spheroid composites were respectively obtained in the same manner as above, using substrates that were produced in the same manner as in Examples 2 to 9, instead of the substrate produced in Example 1.

In each of the spheroid composites obtained in Example 1 to Example 11, plural spheroids having a uniform size were formed a porous base material.
Further, when the obtained spheroid composites were assessed with respect to death/survival using MTT staining as described below, it was confirmed that the spheroids in the spheroid composites were living while maintaining a uniform size.

### (MTT staining)

A 0.5 mg/ml solution of MTT (3-(4,5-dimethyl-2-thiazolyl)-2,5-diphenyltetrazolium bromide, manufactured by Wako Pure Chemical Industries, Ltd.) (Memα: manufactured by GIBCO) was prepared.
2 ml of the MTT solution was added to the spheroid-containing hydrogel set on the bottom face of a 12-well plate, and the hydrogel was incubated for 3 hours.

### (Example 11)

### - Production of multilayer spheroid composite -

The spheroid composite obtained in Example 1 was set on the bottom face of a 12-well plate, DMEM was added as a culture medium, and cultivation was continued for 1 day under culture conditions of 5% CO₂ and 37°C. Two layers were then stacked such that sides from which the temporary supports had been detached contacted each other, thereby producing a multilayer spheroid composite. The cultivation was further continued for 10 days using the multilayer spheroid composite obtained.
Separately, five layers were stacked such that a side from which the temporary support had been detached and the opposite side contacted each other, thereby producing a multilayer spheroid composite. The cultivation was further continued for 10 days using the multilayer spheroid composite obtained.
It was confirmed by MTT staining that the spheroids were living in both multilayer spheroid composites.

Accordingly, it is understood that a spheroid composite including plural spheroids having a uniform size and formed on a porous base material could be formed efficiently according to the method of producing a spheroid composite according to the invention.

### (Reference Example 9)

### - Production of substrate -

The following operations were all performed in a yellow room.
The branched polyalkylene glycol derivative (multi-arm PEG-azide: 4PA20K) prepared in Reference Example 1 was dissolved in toluene to prepare a toluene solution (1%) of 4PA20K as a photosensitive composition A. A poly-L-lysine-coated glass slide (white cut NO. 1 glass slide with unprocessed edges, circular type having a diameter of 21 mm, manufactured by Matsunami Glass Ind., Ltd., hereinafter simply referred to as "PLL-coated glass") was used as a substrate. 110 µL of the photosensitive composition A was dripped on the PLL-coated glass, and a film was formed using a spin coating method (500 rpm × 5 seconds+3,000 rpm × 20 seconds+6,000 rpm × 1 second) and dried by being left to stand at ordinary temperature. The film was brought into close contact with a photomask made of quartz glass (on which a number of circular patterns having a diameter of 100 µm were disposed), and was exposed to light using a high-pressure mercury lamp (200 W) for 40 seconds. Thereafter, the film was washed with deionized water (development step: running water for 15 seconds+immersion for 20 minutes). The film was dried at ordinary temperature, as a result of which a substrate having a microfabricated hydrophilic crosslinked material on its surface was obtained. The surface of the substrate was observed by a phase-difference optical microscope (magnification × 100), and it was confirmed that a fine micropattern was formed at high accuracy.

### (Reference Example 10)

A substrate was produced in the same manner as in Reference Example 9, except that an aminopropylsilane-coated glass (APS-coated NO. 1 cover glass, circular type having a diameter of 21 mm, manufactured by Matsunami Glass Ind., Ltd., hereinafter simply referred to as "APS-coated glass") was used as the substrate instead of the PLL-coated glass in Reference Example 9, whereby a substrate having a microfabricated hydrophilic crosslinked material on its surface was obtained. When the surface of the substrate was observed by a phase-difference optical microscope, it was confirmed that a fine micropattem was formed at high accuracy.

### (Reference Example 11)

A substrate was produced in the same manner as in Reference Example 9, except that an MAS-coated glass (manufactured by Matsunami Glass Ind., Ltd.) was used as the substrate instead of the PLL-coated glass in Reference Example 9, whereby a substrate having a microfabricated hydrophilic crosslinked material on its surface was obtained. When the surface of the substrate was observed by a phase-difference optical microscope, it was confirmed that a fine micropattem was formed at high accuracy.

### (Reference Example 12)

A substrate was produced in the same manner as in Reference Example 9, except that a "collagen-coated glass" in which collagen was further coated on the PLL coating was used as the substrate instead of the PLL-coated glass in Reference Example 9, whereby a substrate having a microfabricated hydrophilic crosslinked material on its surface was obtained. Observation of the substrate surface using a phase-difference optical microscope (magnification × 100) confirmed that a fine micropattem was formed at high accuracy.
The collagen-coated glass was prepared by repeating twice the processes of dripping 400 µL of a 0.1% aqueous solution of swine type-I collagen (manufactured by Nippon Meat Packers, Inc.) on the PLL-coated glass, thereafter forming a film by a spin coating method (350 rpm × 5 seconds+500 rpm × 5 seconds+1,000 rpm × 10 seconds+1,500 rpm × 10 seconds+6,000 rpm × 1 second), and drying the film at room temperature.

### (Reference Example 13)

A substrate was produced in the same manner as in Reference Example 9, except that a "gelatin-coated glass" in which gelatin was further coated on the PLL coating was used as the substrate instead of the PLL-coated glass in Reference Example 9, whereby a substrate having a microfabricated hydrophilic crosslinked material on its surface was obtained. When the surface of the substrate was observed by a phase-difference optical microscope, it was confirmed that a fine micropattem was formed at high accuracy.
The gelatin-coated glass was prepared by dripping 400 µL of a 0.1% solution of gelatin (manufactured by Nitta Gelatin Inc.) on the PLL-coated glass, forming a film by a spin coating method (350 rpm × 5 seconds+500 rpm × 5 seconds+1,000 rpm × 10 seconds+1,500 rpm × 10 seconds+6,000 rpm × 1 second), and thereafter drying the film at room temperature.

### (Reference Example 14)

A substrate was produced in the same manner as in Reference Example 13, except that the method of preparing a collagen-coated glass in Reference Example 13 was changed to a method of immersing the PLL-coated glass in a 0.02% aqueous solution of swine type-I collagen (manufactured by Nippon Meat Packers, Inc.) for 3 hours, washing the glass with running deionized water, and drying the glass. When the surface of the substrate was observed by a phase-difference optical microscope, it was confirmed that a fine micropattem was formed at high accuracy.

### (Reference Example 15)

A substrate was produced in the same manner as in Reference Example 9, except that the photosensitive composition B in which the concentration of the multi-arm PEG-azide (4PA20K) was set to be 0.5% was used instead of the photosensitive composition A in Reference Example 9. When the surface of the substrate was observed by a phase-difference optical microscope, it was confirmed that a fine micropattem was formed at high accuracy.

### (Reference Example 16)

Substrates were produced in the same manner as in Reference Example 9, except that various branched polyalkylene glycol derivatives synthesized in Reference Examples 2 to 5 were respectively used as the branched polyalkylene glycol derivative instead of 4PA20K in Reference Example 9. When the surface of each of the substrates was observed by a phase-difference optical microscope, it was confirmed that a fine micropattern was formed at high accuracy.

### (Reference Example 17)

A substrate was produced in the same manner as in Reference Example 9, except that the branched polyalkylene glycol derivative (4PC20K) synthesized in Reference Example 4 was used instead of the branched polyalkylene glycol derivative (4PA20K) in Reference Example 9 and the light exposure conditions were changed to use of a high-pressure mercury lamp (200 W) for 3 seconds. The surface of the substrate was observed by a phase-difference optical microscope (magnification × 100), and it was confirmed that a fine micropattern was formed at high accuracy.

### (Reference Example 18)

A substrate was produced in the same manner as in Reference Example 9, except that the branched polyalkylene glycol derivative (4PC20K) synthesized in Reference Example 4 was used instead of the branched polyalkylene glycol derivative (4PA20K) in Reference Example 9, and the light exposure conditions were changed to light exposure using a high-pressure mercury lamp (200 W) for 10 seconds with a filter (UTVAF36U, manufactured by Sigma Koki Co., Ltd) disposed on the photomask. The surface of the substrate was observed by a phase-difference optical microscope (magnification × 100), and it was confirmed that a fine micropattern was formed at high accuracy.

### (Reference Example 19)

Substrates were produced in the same manner as in Reference Examples 10 to 14, respectively, except that the branched polyalkylene glycol derivative (4PB20K) synthesized in Reference Example 2 or the branched polyalkylene glycol derivative (4PC20K) synthesized in Reference Example 4 was used instead of the branched polyalkylene glycol derivative (4PA20K) in Reference Examples 10 to 14, and the light exposure conditions were changed to light exposure using a high-pressure mercury lamp (200 W) for 10 seconds with a filter (UTVAF36U, manufactured by Sigma Koki Co., Ltd) disposed on the photomask. The surface of each of the substrates was observed by a phase-difference optical microscope, and it was confirmed that a fine micropattem was formed at high accuracy.

### (Reference Example 20)

A photosensitive composition D was prepared by dissolving the branched polyalkylene glycol derivatives (4PD20K, 8PD20K) prepared in Reference Examples 6 and 7 and IRGACURE 2959 as a photopolymerization initiator in toluene such that the branched polyalkylene glycol derivatives had a concentration of 1% and IRGACURE 2959 had a concentration of 0.05%. A poly-L-lysine-coated glass slide (white cut NO. 1 glass slide with unprocessed edges, circular type having a diameter of 21 mm, manufactured by Matsunami Glass Ind., Ltd., hereinafter simply referred to as "PLL-coated glass") was used as a substrate. 110 µL of the photosensitive composition D was dripped on the PLL-coated glass, and a film was formed using a spin coating method (500 rpm × 5 seconds)+3,000 rpm × 20 seconds+6,000 rpm × 1 second), and dried by being left to stand at ordinary temperature. The film was brought into close contact with a photomask made of quartz glass (on which a number of circular patterns having a diameter of 100 µm were disposed), and was exposed to light using a high-pressure mercury lamp (200 W) for 40 seconds, and thereafter washed with deionized water (development step: running water for 15 seconds+immersion for 20 minutes). The film was dried at ordinary temperature, as a result of which a substrate having a microfabricated hydrophilic crosslinked material on its surface was obtained. The surface of the substrate was observed by a phase-difference optical microscope (magnification × 100), and it was confirmed that a fine micropattem was formed at high accuracy, whichever branched polyalkylene glycol derivative was used.

### (Example 12)

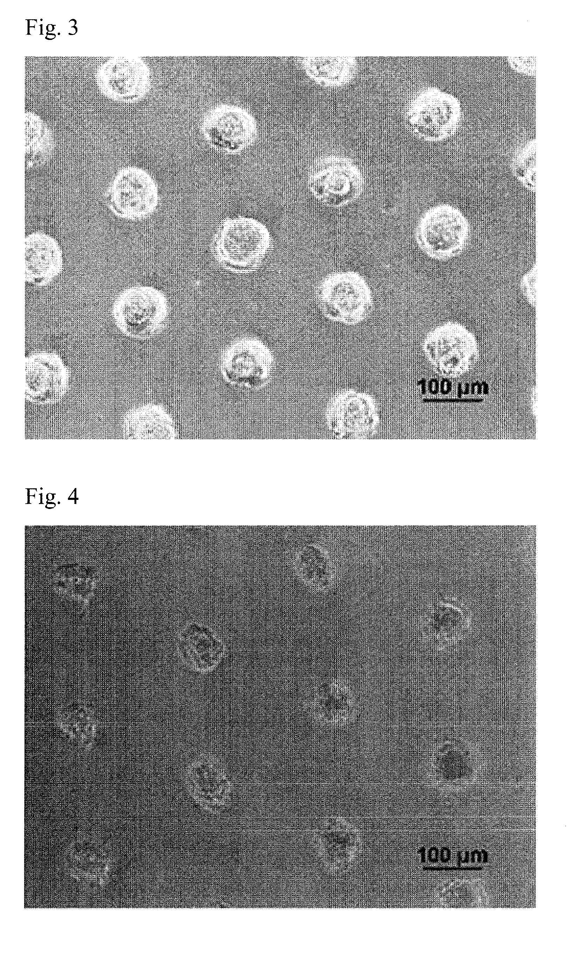

The substrate produced in Reference Example 9 was thereafter sterilized, and was set on the bottom face of a 12-well plate manufactured by FALCON, MEMα (containing 10 vol% of FBS as serum; the culture media used in the following contained serum in the same manner) was added as a culture medium, and osteoblast strain MC3T3-E1 was inoculated thereon at a cell concentration of 1 × 10⁶ cells/mL. The cells were cultured under culture conditions of 5% CO₂ and 37°C, whereby an array of osteoblast spheroids, which had a uniform size and were arranged in a pattern corresponding to the hydrophobic regions formed on the base material, was formed within 24 hours. The appearance of the resultant array of the osteoblast spheroids observed by a phase-difference optical microscope (magnification × 40) is shown in FIG. 3.
Further, in each of the cases in which the substrates produced in other Reference Examples were used in the same manner, an array of osteoblast spheroids, which had a uniform size (about 100 µm) and were arranged in a pattern corresponding to the hydrophobic regions formed on the base material, was formed.

The cultivation was continued for 21 days using the spheroid array culture substrate obtained above (the culture medium was replaced every two days). The spheroid array culture substrate was then taken out, and placed on a Teflon (registered trademark) plate. 140 µl of an MEMα culture medium solution containing 10% of the branched polyalkylene glycol derivative (8PD20K) (the culture medium solution having been passed through a filter of 0.22 µm) was dripped on the culture substrate in the co-presence of a polymerization initiator (IRGACURE2959) at 0.05%. A filter (ultraviolet-transmitting and visible-absorbing filter, UTVAF-50S-36U, manufactured by Sigma Koki Co., Ltd) was then placed on the culture substrate, and the culture substrate was irradiated with a high-pressure mercury lamp (25 mW/cm², 35 seconds) so as to cause gelling and to form a hydrogel composite.
When 1 hour had passed since the formation of the hydrogel composite, the hydrogel was detached from the prepared hydrogel composite by using tweezers, whereby a spheroid-containing hydrogel that contained the spheroids transferred from the substrate was obtained.
The obtained spheroid-containing hydrogel was set on the bottom face of a 12-well plate, MEMα was added as a culture medium, and cultivation was continued for 14 days under culture conditions of 5% CO₂ and 37°C. The state of the spheroid-containing hydrogel on the 8th day of the cultivation is shown in FIG. 4.

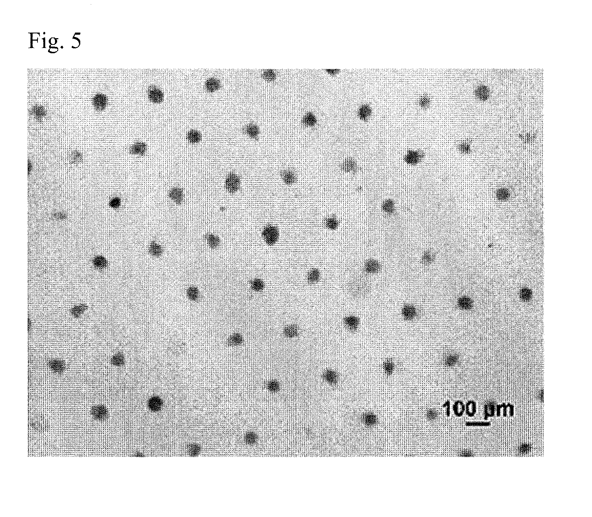

When the obtained spheroid-containing hydrogel was assessed with respect to death/survival using MTT staining as described below, it was confirmed that the spheroids in the spheroid-containing hydrogel were living while maintaining a uniform size (about 100 µm). The MTT-stained spheroid-containing hydrogel is shown in FIG. 5.

### - MTT staining -

A 0.5 mg/ml solution of MTT (3-(4,5-dimethyl-2-thiazolyl)-2,5-diphenyltetrazolium bromide, manufactured by Wako Pure Chemical Industries, Ltd.) (Memα: manufactured by GIBCO) was prepared.
2 ml of the MTT solution was added to the spheroid-containing hydrogel set on the bottom face of the 12-well plate, and the hydrogel was incubated for 3 hours.

### (Example 13)

A spheroid-containing hydrogel was produced in the same manner as in Example 1, except that a culture medium solution containing 4PD20K instead of 8PD20K in Example 12 as the branched polyalkylene glycol derivative was used.
It was similarly confirmed by MTT staining that the spheroids were living while maintaining a uniform size (about 100 µm).

### (Example 14)

A spheroid-containing hydrogel was produced in the same manner as in Example 1, except that a culture medium solution containing 8PD40K instead of 8PD20K in Example 12 as the branched polyalkylene glycol derivative was used.
It was similarly confirmed by MTT staining that the spheroids were living while maintaining a uniform size (about 100 µm).

### (Example 15)

### - Production of spheroid-containing hydrogel laminated body -

The obtained spheroid-containing hydrogel was set on the bottom face of a 12-well plate, MEMα was added as a culture medium, and cultivation was continued for 1 day under culture conditions of 5% CO₂ and 37°C. Two layers were then stacked such that sides from which the substrates had been detached contacted each other, thereby producing a spheroid-containing hydrogel laminated body. The cultivation was further continued for 10 days using the obtained hydrogel laminated body.
Further, five layers were stacked such that a side from which the substrate had been detached and the opposite side contacted each other, thereby producing a hydrogel laminated body. The cultivation was further continued for 10 days using the obtained spheroid-containing hydrogel laminated body.
It was similarly confirmed that the spheroids were living while maintaining a uniform size (about 100 µm) in each of the spheroid-containing hydrogel laminated bodies.

### (Example 16)

The substrate produced in Reference Example 9 was subjected to a sterilization operation, and set on the bottom face of a 12-well plate manufactured by FALCON. DMEM was added as a culture medium, and bovine knee joint cartilage cells were inoculated thereto at a cell concentration of 1 × 10⁶ cells/mL. The cells were cultured under culture conditions of 5% CO₂ and 37°C, whereby an array of bovine knee joint cartilage cell spheroids, which had a uniform size (about 100 µm) and were arranged in a pattern corresponding to the hydrophobic regions formed on the base material, was formed within 24 hours.
Further, in each of the cases in which the substrates produced in other Reference Examples were used in the same manner, an array of bovine knee joint cartilage cell spheroids, which had a uniform size (about 100 µm) and were arranged in a pattern corresponding to the hydrophobic regions formed on the base material, was similarly formed.

The cultivation was continued for 14 days using the spheroid array culture substrate obtained above. The spheroid array culture substrate was then taken out, and placed on a Teflon (registered trademark) plate. 140 µl of a DMEM culture medium solution containing 10% of the branched polyalkylene glycol derivative (8PD40K) (the culture medium solution having been passed through a filter of 0.22 µm) was dripped on the culture substrate in the co-presence of a polymerization initiator (IRGACURE2959) at 0.05%. A filter was then placed on the culture substrate, and the culture substrate was irradiated with a high-pressure mercury lamp (25 mW/cm², 35 seconds) so as to cause gelling and to form a hydrogel.
When 24 hours had passed since the formation of the hydrogel composite, the hydrogel was detached from the hydrogel composite by using tweezers, whereby a spheroid-containing hydrogel that contained the spheroids transferred from the substrate was obtained.
The obtained spheroid-containing hydrogel was set on the bottom face of a 12-well plate, DMEM was added as a culture medium, and cultivation was continued for 14 days under culture conditions of 5% CO₂ and 37°C.

When the obtained spheroid-containing hydrogel was assessed with respect to death/survival using MTT staining as described above, it was confirmed that the spheroids in the spheroid-containing hydrogel were living while maintaining a uniform size (about 100 µm).

### (Example 17)

The substrate produced in Reference Example 9 was subjected to a sterilization operation, and set on the bottom face of a 12-well plate manufactured by FALCON. A Williams'E culture medium was added as a culture medium, and hepatic cells were inoculated at a cell concentration of 1 × 10⁶ cells/mL. When the cells were cultured under culture conditions of 5% CO₂ and 37°C, an array of hepatic cell spheroids, which had a uniform size (about 100 µm) and were arranged in a pattern corresponding to the hydrophobic regions formed on the base material, was formed within 24 hours.
Further, in each of the cases in which the substrates produced in other Reference Examples were used in the same manner, an array of hepatic cell spheroids, which had a uniform size (about 100 µm) and were arranged in a pattern corresponding to the hydrophobic regions formed on the base material, was similarly formed.

The cultivation was continued for 21 days using the spheroid array culture substrate obtained above. The spheroid array culture substrate was then taken out, and placed on a Teflon (registered trademark) plate. 140 µl of a Williams'E culture medium solution containing 10% of the branched polyalkylene glycol derivative (8PD40K) (the culture medium solution having been passed through a filter of 0.22 µm) was dripped on the culture substrate in the co-presence of a polymerization initiator (IRGACURE2959) at 0.05%. A filter was then placed on the culture substrate, and the culture substrate was irradiated with a high-pressure mercury lamp (25 mW/cm², 35 seconds) so as to cause gelling and to form a hydrogel.
When 24 hours had passed since the formation of the hydrogel composite, the hydrogel was detached from the hydrogel composite by using tweezers, whereby a spheroid-containing hydrogel that contained the spheroids transferred from the substrate was obtained.
The obtained spheroid-containing hydrogel was set on the bottom face of a 12-well plate, Williams'E was added as a culture medium, and cultivation was continued for 14 days under culture conditions of 5% CO₂ and 37°C.

When the obtained spheroid-containing hydrogel was assessed with respect to death/survival using MTT staining as described above, it was confirmed that the spheroids in the spheroid-containing hydrogel were living while maintaining a uniform size (about 100 µm).

### (Example 18)

A white cut glass slide (manufactured by Matsunami Glass Ind., Ltd., circular type having a diameter of 21 mm) was washed with ozone (15 minutes × 2 times). The glass slide was immersed in a mixed solution of 1 ml of a coupling agent DATES ((N,N'-diethylamino)dithiocarbamoylpropyl(triethoxy)silane), 8 ml of chloroform, 1 ml of methanol and 85 µl of concentrated hydrochloric acid for 30 minutes. Thereafter the glass slide was dried at 70°C for 30 minutes, washed sequentially with chloroform, methanol and Milli-Q water, and dried in a desiccator under reduced pressure.
The resultant was put in a solid Teflon (registered trademark) container, and the container was filled with a 4 mod/l solution of IPAAm (isopropylacrylamide) in THF that had been bubbled with argon gas for 1 hour. A quartz glass was placed on the glass slide in such a manner that air is not incorporated, and the glass slide was irradiated with UV at 25 W/cm² for 10 minutes. Thereafter the glass slide was washed sequentially with methanol and Milli-Q water, and was dried.
The glass slide was then coated with gelatin by being immersed in a 0.15% aqueous solution of gelatin for 2 hours followed by drying.
Through the above processes, a glass substrate having a temperature-responsive layer was obtained.

A substrate including plural hydrophilic regions and hydrophobic regions formed on a base material was produced in the same manner as in Reference Example 9, except that the glass substrate having a temperature-responsive layer obtained above was used instead of the PLL-coated glass slide in Reference Example 9.
A hydrogel composite was produced in the same manner as in Example 12, except that the thus-produced substrate was used.
When 24 hours had passed since the formation of the hydrogel composite, the hydrogel was detached from the hydrogel composite at a temperature condition of 25°C by using tweezers, whereby a spheroid-containing hydrogel that contained the spheroids transferred from the substrate was obtained.
The obtained spheroid-containing hydrogel was set on the bottom face of a 12-well plate, MEMα was added as a culture medium, and cultivation was continued for 14 days under culture conditions of 5% CO₂ and 37°C.

When the obtained spheroid-containing hydrogel was assessed with respect to death/survival using MTT staining as described above, it was confirmed that the spheroids in the spheroid-containing hydrogel were living while maintaining a uniform size (about 100 µm).
Further, similar results were obtained when a substrate coated with fibronectin or collagen instead of the gelatin coating was used in the above experiment.

### (Example 19)

The substrate produced in Reference Example 9 was subjected to a sterilization operation, and set on the bottom face of a 12-well plate manufactured by FALCON. Using DMEM (containing 10 vol% of FBS as serum) as a culture medium, bovine aorta endothelial cells (BAEC) were inoculated at a cell concentration of 5 × 10⁶ cells/mL (2 mL/well). The cells were cultured under culture conditions of 5% CO₂ and 37°C, as a result of which the cells arranged themselves in a pattern corresponding to the hydrophobic regions formed on the base material within 24 hours.
Using Williams'E (containing 10 vol% of FBS as serum) as a culture medium, rat primary hepatic cells were inoculated, at a cell concentration of 5 × 10₆ cells/mL (2 mL/well), onto this substrate on which BAECs had been cultured in pattern. The cells were cultured under culture conditions of 5% CO₂ and 37°C, and hepatic cell aggregates (spheroids) having a uniform size were obtained on the patterned BAECs within 24 hours.
Using this substrate on which the spheroids had been formed, a spheroid-containing hydrogel was produced in the same manner as in Example 12.
The obtained spheroid-containing hydrogel was set on the bottom face of a 12-well plate, Williams'E was added as a culture medium, and cultivation was continued under culture conditions of 5% CO₂ and 37°C for 14 days.

When the obtained spheroid-containing hydrogel was assessed with respect to death/survival using MTT staining as described above, it was confirmed that the spheroids in the spheroid-containing hydrogel were living while maintaining a uniform size (about 100 µm).

### (Example 20)

The patterned substrate was subjected to sterilization treatment, and set on the bottom face of a 12-well plate manufactured by FALCON. Using DMEM (containing 10 vol% of FBS as serum) as a culture medium, murine fibroblasts (NIH-3T3) were inoculated at a cell concentration of 5 × 10⁶ cells/mL (2 mL/well). When the cells were cultured under culture conditions of 5% CO₂ and 37°C, the cells arranged themselves in a pattern corresponding fin the hydrophobic regions formed on the base material were obtained within 24 hours. Using Williams`E (containing 10 vol% of FBC as serum) as a culture medium, rat primary hepatic cells were inoculated at a cell concentration of 5 × 10⁶ cells/mL (2 mL/well) onto this substrate on which NIH-3T3 cells had been cultured in pattern. The cells were cultured under culture conditions of 5% CO₂ and 37°C, and hepatic cell aggregates (spheroids) having a uniform size were obtained on the patterned NIH-3T3 cells within 24 hours.
Using this substrate on which the spheroids had been formed, a spheroid-containing hydrogel was produced in the same manner as in Example 12.
The obtained spheroid-containing hydrogel was set on the bottom face of a 12-well plate, Williams'E was added as a culture medium, and cultivation was continued under culture conditions of 5% CO₂ and 37°C for 14 days.

When the obtained spheroid-containing hydrogel was assessed with respect to death/survival using MTT staining as described above, it was confirmed that the spheroids in the spheroid-containing hydrogel were living while maintaining a uniform size (about 100 µm).

### (Example 21)

A substrate was produced using a quartz glass photomask having a circular pattern with a diameter of 200 µm instead of the quartz glass photomask having a circular pattern with a diameter of 100 µm in the production of the substrate in Reference Example 9. Using this substrate, a spheroid-containing hydrogel was produced in the same manner as in Example 12.
When the obtained spheroid-containing hydrogel was assessed with respect to death/survival using MTT staining as described above, it was confirmed that the spheroids in the spheroid-containing hydrogel were living while maintaining a uniform size (about 200 µm).

### (Example 22)

A substrate was produced using a quartz glass photomask having a circular pattern with a diameter of 70 µm instead of the quartz glass photomask having a circular pattern with a diameter of 100 µm in the production of the substrate in Reference Example 9. Using this substrate, a spheroid-containing hydrogel was produced in the same manner as in Example 12.
When the obtained spheroid-containing hydrogel was assessed with respect to death/survival Using MTT staining as described above, it was confirmed that the spheroids in the spheroid-containing hydrogel were living while maintaining a uniform size (about 70 µm).

### (Comparative Example 1)

A substrate was produced using a quartz glass photomask having a circular pattern with a diameter of 500 µm instead of the quartz glass photomask having a circular pattern with a diameter of 100 µm in the production of the substrate in Reference Example 9. Using this substrate, a spheroid-containing hydrogel was produced in the same manner as in Example 12.
When the obtained spheroid-containing hydrogel was assessed with respect to death/survival using MTT staining as described above, it was confirmed that the spheroids (size: about 500 µm) in the spheroid-containing hydrogel had extremely low viability.

### (Comparative Example 2)

A substrate was produced using a quartz glass photomask having a circular pattern with a diameter of 50 µm instead of the quartz glass photomask having a circular pattern with a diameter of 100 µm in the production of the substrate in Reference Example 9. Using this substrate, a spheroid-containing hydrogel was produced in the same manner as in Example 12.
When the obtained spheroid-containing hydrogel was assessed with respect to death/survival using MTT staining as described above, it was confirmed that the spheroids (size: about 50 µm) in the spheroid-containing hydrogel had poor cell aggregation properties and extremely low viability.

Accordingly, it is understood that a spheroid-containing hydrogel including plural spheroids having a uniform size could be produced by the method of producing a spheroid-containing hydrogel according to the invention.
Further, it is also understood that the function-maintainability of the spheroids is improved by adjusting the size of the spheroids in the hydrogel to fall within a specific range.

## Claims

1. A spheroid composite comprising:
a substrate comprising:
a cell-adhesive porous base material; and
a plurality of hydrophilic regions and hydrophobic regions that are disposed on the porous base material and are formed by curing a photosensitive composition, wherein the photosensitive composition includes a branched polyalkylene glycol derivative having:
3 or more polyalkylene glycol groups, each having a polymerizable substituent at a terminal thereof; and
a tri- or higher-valent linking group that binds to the polyalkylene glycol groups; and
spheroids formed in the hydrophobic regions on the substrate.

2. The spheroid composite according to claim 1, wherein the branched polyalkylene glycol derivative has 4 or more polyalkylene glycol groups that have polymerization degrees of from 5 to 1,000.

3. The spheroid composite according to claim 1 or 2, wherein the porous base material comprises at least one of a biodegradable compound or an extracellular matrix.

4. The spheroid composite according to any one of claims 1 to 3, wherein the porous base material comprises at least one selected from polyglycolic acid, polylactic acid, poly(ε-caprolactone), gelatin, collagen, alginic acid, fibrin, an adhesion protein derived from lectin, polylysine or an adhesive oligopeptide.

5. The spheroid composite according to any one of claims 1 to 4, wherein the hydrophobic regions are formed in an array on the porous base material.

6. A multilayer spheroid composite comprising two or more of the spheroid composite according to any one of claims 1 to 5, which are disposed one on another in layers.

7. A method of producing a spheroid composite, the method comprising:
inoculating cells onto a substrate comprising:
a cell-adhesive porous base material; and
a plurality of hydrophilic regions and hydrophobic regions that are disposed on the porous base material and are formed by curing a photosensitive composition, wherein the photosensitive composition includes a branched polyalkylene glycol derivative having:
3 or more polyalkylene glycol groups, each having a polymerizable substituent at a terminal thereof; and
a tri- or higher-valent linking group that binds to the polyalkylene glycol groups; and
culturing the inoculated cells to form cultured-cell-derived spheroids in the hydrophobic regions on the substrate.

8. The method of producing a spheroid composite according to claim 7, wherein the branched polyalkylene glycol derivative has 4 or more polyalkylene glycol groups that have polymerization degrees of from 5 to 1,000.

9. The method of producing a spheroid composite according to claim 7 or 8, wherein the porous base material comprises at least one of a biodegradable compound or an extracellular matrix.

10. The method of producing a spheroid composite according to any one of claims 7 to 9, wherein the porous base material comprises at least one selected from polyglycolic acid, polylactic acid, poly(ε-caprolactone), gelatin, collagen, alginic acid, fibrin, an adhesion protein derived from lectin, polylysine or an adhesive oligopeptide.

11. The method of producing a spheroid composite according to any one of claims 7 to 10, wherein the porous base material is disposed on a temperature-responsive layer, characteristics of which change in a temperature-responsive manner.

12. The method of producing a spheroid composite according to any one of claims 7 to 11, wherein the hydrophobic regions are formed in an array on the porous base material.

13. A spheroid-containing hydrogel, comprising:
a hydrogel; and
two or more spheroids that have a uniform size with a diameter of from 70 µm to 400 µm, and that are disposed in the hydrogel in such a manner that the two or more spheroids do not contact each other.

14. The spheroid-containing hydrogel according to claim 13, wherein the hydrogel comprises a polymer compound derived from a macromonomer for forming a hydrogel, and wherein the macromonomer has a weight average molecular weight of 10,000 or more, has 4 or more polyalkylene glycol groups, each having a polymerizable substituent at a terminal thereof, and has a tetra- or higher-valent linking group that binds to the polyalkylene glycol groups.

15. The spheroid-containing hydrogel according to claim 14, wherein the macromonomer for forming a hydrogel has 4 or more polyalkylene glycol groups that have polymerization degrees of from 50 to 5,000.

16. The spheroid-containing hydrogel according to claim 14 or 15, wherein the polymerizable substituent has an ethylenic unsaturated bond.

17. A spheroid-containing hydrogel laminated body comprising two or more of the spheroid-containing hydrogel according to any one of claims 13 to 16, which are disposed one on another in layers.

18. A method of producing a spheroid-containing hydrogel, the method comprising:
a step of inoculating cells onto a substrate comprising a base material and a plurality of hydrophilic regions and hydrophobic regions that are formed on the base material;
a spheroid formation step of culturing the inoculated cells to form cultured-cell-derived spheroids in the hydrophobic regions on the substrate;
a hydrogel-composite formation step of disposing a hydrogel on a side of the substrate, on which the spheroids are formed, to form a hydrogel composite; and
a step of detaching the substrate from the hydrogel composite to obtain a spheroid-containing hydrogel.

19. The method of producing a spheroid-containing hydrogel according to claim 18, wherein the hydrogel-composite formation step comprises:
a step of disposing a photosensitive composition comprising a hydrophilic polymer compound on a side of the substrate on which the spheroids are formed, so as to contact the spheroids with the photosensitive composition; and
a step of curing the photosensitive composition.

20. The method of producing a spheroid-containing hydrogel according to claim 19, wherein the photosensitive composition comprises a macromonomer for forming a hydrogel, and wherein the macromonomer has a weight average molecular weight of 10,000 or more, has 4 or more polyalkylene glycol groups, each having a polymerizable substituent at a terminal thereof, and has a tetra- or higher-valent linking group that binds to the polyalkylene glycol groups.

21. The method of producing a spheroid-containing hydrogel according to claim 20, wherein the macromonomer for forming a hydrogel has 4 or more polyalkylene glycol groups that have polymerization degrees of from 50 to 5,000.

22. The method of producing a spheroid-containing hydrogel according to claim 20 or 21, wherein the polymerizable substituent has an ethylenic unsaturated bond.

23. The method of producing a spheroid-containing hydrogel according to any one of claims 18 to 22, wherein the culturing of the cells in the spheroid formation step is conducted for at least 5 days.

24. The method of producing a spheroid-containing hydrogel according to any one of claims 18 to 23, wherein the hydrophilic regions on the base material comprise a polymer derived from a branched polyalkylene glycol derivative having 3 or more polyalkylene glycol groups, each having a polymerizable substituent at a terminal thereof, and a tri- or higher-valent linking group that binds to the polyalkylene glycol groups.

25. The method of producing a spheroid-containing hydrogel according to any one of claims 18 to 24, wherein the hydrophilic regions are formed on a temperature-responsive layer.

26. The method of producing a spheroid-containing hydrogel according to any one of claims 18 to 25, wherein the hydrophobic regions are formed in an array on the base material.
